# EUROPEAN PATENT SPECIFICATION

(11) **EP 3 508 554 B1**
(45) Date of publication and mention of the grant of the patent: **20.01.2021**
(21) Application number: 17912346.8
(22) Date of filing: 21.12.2017
(51) Int. Cl.: C09K 9/02, C09K 11/02, C09D 5/22, C07D 491/107, C07D 311/94, G02B 1/04, G02B 1/14, G02C 7/10, B01J 13/14, C09K 11/56

(54) **PHOTOCHROMIC OPTICAL MATERIAL AND PREPARATION METHOD THEREFOR**
PHOTOCHROMES OPTISCHES MATERIAL UND HERSTELLUNGSVERFAHREN DAFÜR
MATÉRIAU OPTIQUE PHOTOCHROMIQUE ET PROCÉDÉ DE PRÉPARATION ASSOCIÉ

(30) Priority: 10.11.2017 CN 201711113657; 10.11.2017 CN 201711110840; 10.11.2017 CN 201711110839; 10.11.2017 CN 201711110838; 10.11.2017 CN 201711112631; 10.11.2017 CN 201711110837
(43) Date of publication of application: 10.07.2019
(73) Proprietor: Jiangsu Shikexincai Company Limited, Jinhu County Economic Development Zone Huai'an Jiangsu 211600 (CN)
(72) Inventor: WANG, Minghua, Huai'an Jiangsu 211600 (CN); QIAO, Zhenan, Huai'an Jiangsu 211600 (CN); ZHANG, Hejun, Huai'an Jiangsu 211600 (CN); JI, Lijun, Huai'an Jiangsu 211600 (CN); FAN, Weizheng, Huai'an Jiangsu 211600 (CN); ZHANG, Qian, Huai'an Jiangsu 211600 (CN); SI, Yunfeng, Huai'an Jiangsu 211600 (CN); WANG, Zhifei, Huai'an Jiangsu 211600 (CN); LIU, Yang, Huai'an Jiangsu 211600 (CN); XUE, Xiaohua, Huai'an Jiangsu 211600 (CN); WU, Xiao, Huai'an Jiangsu 211600 (CN); ZHENG, Yonghua, Huai'an Jiangsu 211600 (CN)
(74) Representative: Cabinet Beau de Loménie
(86) International application number: PCT/CN2017/117714
(87) International publication number: WO 2019/090920

(56) References cited:
- EP-A1- 0 346 484
- WO-A1-2017/038957
- CN-A- 101 225 296
- CN-A- 103 215 029
- CN-A- 103 962 076
- CN-A- 103 962 076
- KR-A- 20090 124 431
- KR-A- 20090 124 431

## Description

### Technical Field

The invention belongs to the field of photochromic technique, and specifically relates to a photochromic nano-composite microsphere, as well as the paint, coating and photochromic optical material prepared therefrom.

### Background Art

Photochromism means that when certain compound is under the action of the light of certain wavelength and intensity, the molecular structure of the material changes with the change of the absorption spectra, leading to the reversible change of the absorbance and color of the material, and when the light is stopped the functional material can automatically recovers back to the original state, and as a novel material of the sub-field of optical material, it has an important value of application in the high technology of optical lens, optical information storage, molecular switches, and forgery-proof identification etc. The photochromic phenomenon has been found by the human being for more than one hundred years. The first successful commercial application began in the 1960s, two material scientists of Corning working lab of the United States - Amistead and Stooky - first prepared the photochromic material for the silver halide-containing glass, later on lots of studies were carried out on its mechanism and application, and novel photochromic glasses were developed. However, due to their relatively high cost and complex manufacturing technique, they are not suitable to make the large area phototropic glass, and their commercial application in the construction field is limited. Thereafter, the application focus of photochromism shifted to the polymer-based material with low price and light weight; the photochromic compound was added to a clear resin to make the photochromic material, which can be used in the resin lenses, and has been applied in the photochromic glasses both at home and abroad. The silver halide photochromic material has gradually phased out because it is not suitable for the resin glasses. Presently the application of the organic photochromic compound in the resin lens mainly comprises the following categories: spiroindoline naphthooxazine, naphthopyran, and fulgide photochromic compound, etc. Although important results have been achieved in the practical aspect, disadvantages of the photochromic compound including poor photo-stability, low photochromic response value, and insufficient thermal stability, etc. still exist, for example: the photochromic compound is readily affected by the pH value, oxidation, light intensity, temperature, substrate and environment, etc., resulting in the deterioration of its photochemical fatigue degree, and degradation and deterioration occurs after multiple repeated irradiations and the reversible photochromic property is lost, or the cases such as the photochromic response time is extended, the color fades, and the fading recovery time is slow.

The photo-stability of the photochromic compound, i.e., the fatigue resistance, is one of the important problems existing in the practical application of the photochromic compound. Studies of the photochromic mechanism indicate that the reason leading to fatigue is mainly due to the photo-oxidative degradation occurring when the compound undergoes the photo-isomerization, and the fatigue phenomenon appears. Therefore, the antioxidation study has become the main approach to improve the fatigue resistance of the compound. Currently, the following methods are mainly used to improve the fatigue resistance: the first method is to add the light stabilizer or the antioxidant into the polymer medium containing the photochromic compound; the second method is to bond the antioxidation group onto the compound molecule; the third method is to introduce the electro-rich heteroaryl group into the side chain of the compound molecule; the fourth method is to introduce the singlet oxygen quencher, or to use the spin-trapping agent (spin-trapping agent) to improve the fatigue resistance. Therefore, some investigators improved the performance by microencapsulation of the photochromic compound, for example: patent ZL200810057392 discloses a preparation method of the photochromic microencapsule, and the method can improve the acid-alkali resistance and the fatigue resistance of the photochromic material, and extend the operation life. Patent ZL201410195430.3 discloses a preparation method of the polyurethane-chitosan double-shell photochromic microencapsule, and the double-layer coating can further improve the coating rates and the fatigue resistance, etc. of the photochromic compound. Therefore, To develop the compound and the composite material having good photochromic properties has become one of the research focuses of the photochromic material, especially to develope the photochromic material having excellent fatigue resistance, good thermal stability, and high spectral response value, which is worth more to be promoted and used.

In addition, the photochromic property is also often affected by the polymer molecule, the other chemical adjuvants in the substrate, the microstructure, and the polymerization environment, etc., for example: the impact of the UV absorbent in the polymer to the photochromic compound is relatively big, because the UV light is absorbed by the absorbent in the polymer, the color change of the photochromic compound under the UV light excitation is substantially limited, resulting in low photochromic efficiency; meanwhile, the existence of the diopter results in the inconsistence of the thickness of the edge of the lens with the thickness of the center, and in this case, if the method to add the photochromic compound as a bulk is still used, the color difference of the color depth at positions of the lens of different thickness will occur. Therefore, some of the domestic manufacturers use the spin-coating approach to produce the photochromic lens. The spin-coating approach is to add the photochromic solution formulated into the tank of the spin coater after the substrate is made, and after the mixture is sufficiently mixed the substrate is fixed on the spin coater to conduct the centrifuge and rotation movement and cured to form the particle having the photochromic effect; compared with the substrate approach, the advantage of the spin-coating approach is the high technical content, theoretically capable of being made into any product, and small color difference of the product, and the disadvantage is that the photochromic space decreases and changes with the external environment temperature, and the photochromic efficiency is not high due to the relatively small photochromic coating space; therefore, to develop the lens coating technology with high photochromic efficiency is an important development direction in the future.

### Summary of the Invention

The purpose of the invention is to provide a polyurethane/spiropyran/zinc sulfide photochromic nano-composite microsphere, as well as the paint, coating and photochromic optical material prepared therefrom. The nano-composite microsphere is a composite multi-layer core-shell structure, its core is a sodium acetate/zinc sulfide nanoparticle, its shell is polyurethane, and spiropyrans photochromic compound being the middle layer between the core and the shell. This material can be made into the photochromic coating, or made into the photochromic material by doping into the resin optical material. This material can become colored from colorless under UV light irradiation, and fades rapidly to colorless after the UV light disappears, and has the advantage of strong fatigue resistance, good stability, and high color changing speed, etc.

To achieve the above purpose, the invention provides the following technical solution:
A photochromic nano-composite microsphere is a polyurethane/spiropyran/zinc sulfide three-layer composite core-shell structure: its core is a zinc sulfide mesoporous nano-microsphere, its middle layer is a photochromic layer composed of the spiropyrans compound, and its shell is polyurethane; the outer diameter of the composite microsphere is 50 - 350 nm, wherein, the diameter of the zinc sulfide mesoporous nano-microsphere is 30 ∼ 250 nm, the thickness of the middle layer is 5 ∼ 25 nm, and the thickness of the shell is 5 ∼ 25 nm.

The photochromic nano-composite microsphere described above, preferably the zinc sulfide mesoporous nano-microsphere, is a monodispersed zinc sulfide mesoporous nanosphere consisting of zinc sulfide nano-crystalline, and the middle layer is a nano-composite mesoporous structure formed by uniformly dispersing the spiropyrans compound nanoparticle on the surface or in the pore of the zinc sulfide mesoporous nanosphere.

The photochromic nano-composite microsphere described above, preferably the spiropyrans compound is selected from the compounds of formula (I), formula (II) or formula (III): 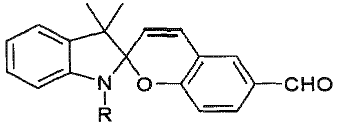 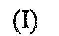 wherein R = C₁₆H₃₃ 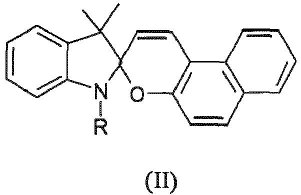 wherein R = C₁₆H₃₃

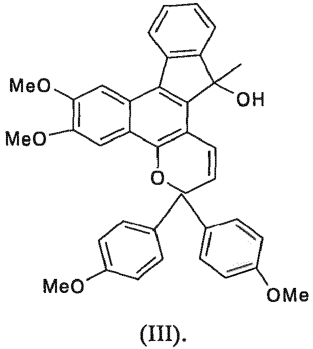

The photochromic nano-composite microsphere above, preferably the polyurethane is obtained by polymerization of an isocyanate monomer compound having two or more isocyanate groups with alcohols compound.

The photochromic nano-composite microsphere described above, preferably the isocyanate monomer compound, is selected from: at least one of toluene diisocyanate, diphenylmethane-4,4'-diisocyanate, 1,6-hexamethylene diisocyanate, m-xylylene diisocyanate, 1,5-naphthalene diisocyanate, methylcyclohexyl diisocyanate, dicyclohexylmethanediisocyanate, tetramethylxylylene diisocyanate, and isophorone diisocyanate;

The alcohols compound is at least one of n-butanol, polytetramethylene ether glycol, pentaerythritol, ethylene glycol, propylene glycol, butylene glycol, hexylene glycol, neopentyl glycol, and trihydroxymethyl propane.

In another aspect, the invention provides a preparation method of the photochromic nano-composite microsphere described above, the method including the following steps:
a. Preparation of the spiropyrans-coated zinc sulfide nanosphere:
   To ethylene glycol is added zinc nitrate, after the mixture is uniformly stirred sulfur powder is added, the temperature is increased to 140-160 °C, and the mixture is reacted for 20-24 hours; the mixture is cooled to 40-60 °C, the mercaptoethanol stabilizer is dropwise added and the mixture is stirred; the spiropyrans compound is added, the mixture is vigorously stirred for 20-40 minutes and cooled to room temperature, the reaction solution gradually separates into layers and precipitates, and the precipitate is collected by filtration, washed, and dried to give the spiropyrans-coated zinc sulfide mesoporous nanosphere; wherein, the mass ratio of zinc nitrate to sulfur powder to mercaptoethanol to ethylene glycol is (0.3-0.6):(0.1-0.2):(4-7):(90-110), and the mass ratio of zinc nitrate to the spiropyrans compound is 1:(0.5-1);
b. Preparation of the polyurethane/spiropyran/zinc sulfide photochromic nano-composite microsphere:
   The spiropyrans-coated zinc sulfide mesoporous nanosphere and the isocyanate monomer compound prepared in step a are added into solvent, after the mixture is uniformly stirred the alcohols compound and a dispersing agent are sequentially added; wherein the mass ratio of the spiropyrans-coated zinc sulfide mesoporous nanosphere to the isocyanate monomer compound to the alcohols compound to the dispersing agent to the solvent is: (0.5-1.2):(0.5-1.2):(0.5-0.9):(1.2-2.2):100; the temperature is kept at 15-25 °C, and the mixture is prepolymerized for 45-60 minutes with stirring to give a prepolymer solution; chain extender and catalyst are added into the prepolymer solution with a mass ratio of the chain extender to the catalyst to the isocyanate monomer compound of (6-15):(0.1-0.3):(9-11), the mixture is heated to 45-90 °C with stirring to conduct chain extending polymerization reaction for 10-15 minutes to give a polyurethane coating substance precipitate, and the precipitate is filtered, washed, and dried to give the photochromic nano-composite microsphere having the polyurethane/spiropyran/zinc sulfide three-layer composite core-shell structure.

Preparation method of the photochromic nano-composite microsphere described above, wherein the solvent is preferably at least one of chloroform, acetone, propyl acetate, butyl acetate, ethyl acetate, dibutyl phthalate, and petroleum ether; the catalyst is dibutyltin dilaurate.

Preparation method of the photochromic nano-composite microsphere described above, the alcohols compound is preferably a mixture of n-butanol and polytetramethylene ether glycol, with a mass ratio of n-butanol to polytetramethylene ether glycol of (1-3):(9-11).

Preparation method of the photochromic nano-composite microsphere described above, the dispersing agent is preferably at least one of Tween 20, Tween 80, Span 20, Span 60, and Span 80;

The chain extender is at least one of ethylene glycol, propylene glycol, ethylene diamine, propylene diamine, and diethylamine;

Preferably, the chain extender is a mixture of ethylene glycol and ethylene diamine with a mass ratio of ethylene glycol to ethylene diamine of (2-3):(0.6-1.5).

In still another aspect, the invention provides a photochromic nano-composite microsphere, which is prepared by the method described above.

In still another aspect, the invention provides a photochromic coating liquid, which comprises the photochromic nano-composite microsphere described above.

The photochromic coating liquid described above, wherein the coating liquid is preferably composed of Component A and Component B according to the parts and weight below:
(1) Component A:
   Polythiocarbamate monomer 60-75 polyurethane/spiropyran/zinc sulfide photochromic nano-composite microsphere 2-7 diluent 7-15
   wetting dispersing agent 1-2
   leveling agent 0-1
(2) Component B:
   isocyanate type curing agent 12-17
   catalyst 0-1
   defoamer 0-1.
   The photochromic coating liquid described above, wherein the Polythiocarbamate monomer is preferably at least one of 2,2'-dimercaptodiethylsulfide, 2,2'-dimercaptoethylthioethane, 2,3-dimercaptoethylthiopropanethiol or 1,2,3-trimercaptoethyl- thiopropane;
   The photochromic coating liquid described above, wherein the isocyanate type curing agent is preferably at least one of toluene diisocyanate curing agent, diphenylmethane-4,4' -diisocyanate curing agent, 1,6-hexamethylene diisocyanate curing agent, m-xylylene diisocyanate curing agent, methylcyclohexyl diisocyanate curing agent, and isophorone diisocyanate curing agent;
   The photochromic coating liquid described above, wherein the catalyst is preferably dibutyltin dilaurate.
   The photochromic coating liquid described above, wherein the diluent is preferably at least one of dichloromethane, butyl acetate, methyl acetate, ethanol, butanol, acetone, toluene, xylene, ethyl ether, and polytetramethylene ether glycol;
   The photochromic coating liquid described above, wherein the wetting dispersing agent is preferably Type BYK-polyurethane exclusive wetting dispersing agent or Type F420 wetting dispersing agent;
   The photochromic coating liquid described above, wherein the leveling agent is preferably Type F300 oily leveling agent, Type F309 leveling agent, Type F309 leveling agent or Type F320 leveling agent;
   The photochromic coating liquid described above, wherein the defoamer is preferably Type XPJ01F modified silicon oil defoamer.

In still another aspect, the invention provides a preparation method of the photochromic coating liquid described above, and this preparation method comprises the following operation steps:
(1) Component A: the Polythiocarbamate monomer, the diluent, the polyurethane/spiropyran/zinc sulfide photochromic composite microsphere, the wetting dispersing agent, and the leveling agent are added into a container respectively according to the ratio described, and the mixture is uniformly stirred at room temperature for 15-30 minutes to give Component A;
(2) Component B: the isocyanate type curing agent, the defoamer, and the catalyst are added into a Container according to the ratio described, and the mixture is uniformly stirred at room temperature for 3-5 minutes to give Component B.

In still another aspect, the invention provides a photochromic coating, which comprises the photochromic nano-composite microsphere described above.

In still another aspect, the invention provides a preparation method of a photochromic coating, the method including: the photochromic coating liquid described above is used to prepare the photochromic coating of optical substrate, the Component A and Component B are uniformly mixed according to the mass ratio of (70-100):(12-19), the viscosity thereof is adjusted by addition of a diluent; the mixture is then coated on the surface of an optical substrate to form a coating layer, which is cured through the light irradiation or heating to give the photochromic coating.

In still another aspect, the invention provides a photochromic optical material, which is composed of an optical resin substrate and the photochromic coating described above coated on the surface thereof.

The photochromic optical material described above, wherein the optical resin substrate is preferably a thermoset plastic or a thermoplastic plastic.

The photochromic optical material described above, wherein the optical resin substrate is preferably one of the polymethyl methacrylate resins, the propenyldiglycol carbonate resins, the polycarbonate resins, the carbamate resins, and the thioepoxy resins.

The photochromic optical material described above, wherein the optical resin substrate is preferably the resin lens.

In still another aspect, the invention provides a preparation method of the photochromic optical material described above, the method including the following steps: the photochromic coating liquid is coated on the optical resin substrate by means of spin-coating, spray-coating or dip-coating to form the optical material having a photochromic coating on the surface.

Preparation method of the photochromic optical material described above, wherein the method preferably includes the following steps:
I. The coating liquid of Component A and Component B is formulated according to the method described above;
II. When coated, Component A and Component B are uniformly mixed according to the mass ratio of (70-100):(12-19), with the viscosity adjusted to 20 ∼ 350 cp (25 °C) by addition of the diluent, to give the photochromic coating liquid;
III. Pretreatment of the optical substrate: one or more of the following pretreatments are conducted to the optical resin substrate: chemical treatment with a basic aqueous solution or an acidic aqueous solution, grinding treatment, plasma treatment under different air pressures, corona discharge treatment, UV ozone treatment, and hardening treatment;
IV. Coating: the optical resin substrate after the pretreatment is spin-coated, spray-coated or dip-coated with the photochromic coating liquid prepared in step II to form a layer of photochromic coating on the substrate, i.e., the spiropyrans photochromic optical material is obtained.

Preparation method of the photochromic optical material, wherein a first photochromic coating liquid having a viscosity of 60 ∼ 250 cp and a second photochromic coating liquid having a viscosity of 50 ∼ 200 cp are prepared respectively preferably in the step II;

The dip-coating method is used in the step IV, and the specific operation thereof is as follow: the optical resin substrate after the pretreatment is immerged into the first photochromic coating liquid, soaked at room temperature for 3-5 minutes, the optical resin substrate is then slowly pulled out of the coating liquid, transferred into an oven set at a temperature of 35-65 °C, and baked for 45-90 minutes to form the first photochromic coating; the optical resin substrate is then immerged into the second photochromic liquid, and soaked at room temperature for 3-5 minutes, the lens is then slowly pulled out of the coating liquid, transferred into an oven set at a temperature of 35-65 °C, and baked for 45-90 minutes, to form the second photochromic coating, and obtain the photochromic optical material. Preparation method of the photochromic optical material, wherein the overall thickness of the photochromic coating is preferably 5 ∼ 100 µm.

Preparation method of the photochromic optical material, wherein the method preferably can also include the hardening and/or antireflective surface coating treatment of the surface of the optical material after the coating step IV.

The spiropyrans photochromic compound described in the invention can be any of the known spiropyrans photochromic compounds, preferably the photochromic spiropyran compound (I), the photochromic spiropyran compound (II), and the photochromic spiropyran compound (III), and these three compounds can be synthesized using the following (but not limited to) method.

### (1) Synthesis of the photochromic spiropyran compound (I):

I. 2,3,3-Trimentylindole and iodohexadecane are added into the chloroform solvent equipped with a refluxing device, wherein the mass ratio of 2,3,3-trimentylindole to iodohexadecane to chloroform is (25-35):(80-110):(350-400), the mixture is heated and refluxed at atmospheric pressure for 16-24 hours, and after the solvent is evaporated and removed, anhydrous ethyl ether is added, leading to the appearance of large amount of light yellow solid, which is suction filtered, sufficiently washed with ethyl ether, and vacuum dried to give hexadecane-2,3,3-trimentylindole (PS01);
II. PS01 is dispersed in water with a mass ratio of water to iodohexadecane of (1-2):1, a 25-35% NaOH solution is dropwise added with continual stirring, until the solid completely disappears, the dropwise addition is stopped, and a light yellow oily sticky material is generated on the liquid surface, together with a clear lower layer, which is extracted with ethyl ether, washed with water, filtered, and rotavaped to remove ethyl ether to give the a yellow clear liquid 3,3-dimethyl-1'-hexadecyl-2-methylene indole (PS02);
III. The ethanol solution containing PS02 and the ethanol solution containing 4-hydroxy-benzene-1,3-dialdehyde are formulated respectively, and they are mixed, wherein the mass ratio of PS02 to ethanol is (1-2):(3-9), the mass ratio of the ethanol solution of PS02 to 4-hydroxy-benzene-1,3-dialdehyde is (2-3):1, and the mass ratio of 4-hydroxy-benzene-1,3-dialdehyde to ethanol is (2-3):(50-100); under protection of nitrogen the ethanol solution of 4-hydroxy-benzene-1,3-dialdehydeis is heated with an oil bath to 45-78 °C to reflux, to the solution is dropwise added a freshly made ethanol solution containing PS02, wherein the reaction solution becomes purple rapidly, followed by continual heating and reflux for 5-6 hours, after the mixture is cooled to room temperature the reaction solution is poured into an ice and stirred to give a light purple emulsion, after the emulsion is left standing sufficiently, water and ethanol are evaporated and removed, and the sticky material is recrystallized with methanol, suction filtered, and dried to give a light purple solid powder, i.e. the photochromic alkyl spiropyrans compound (I).

The specific reaction formula is as follow:

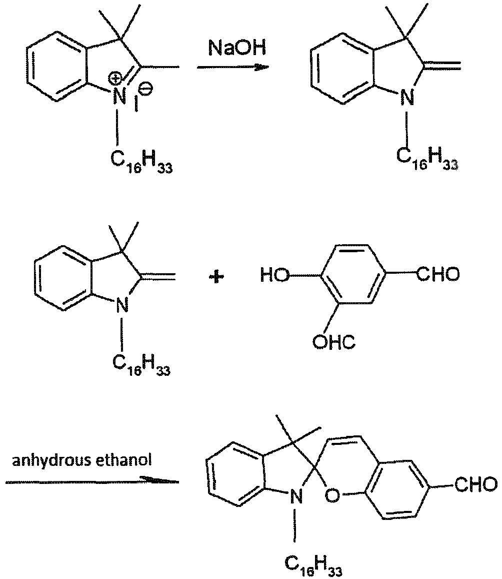

### (2) Synthesis of the photochromic spiropyran compound (II):

2-Hydroxy-1-naphthaldehyde is dissolved in anhydrous ethanol with a mass ratio of (0.8-1.2):(15-30), under the protection of nitrogen the mixture is heated to 45-78 °C and refluxed, an ethanol solution containing PS02 is dropwise added to the mixture, the mass ratio of the ethanol solution of PS02 to 2-hydroxy-1-naphthaldehyde is (2-3):1, wherein the mass ratio of PS02 to anhydrous ethanol is (1-2):(3-9), the mixture is continually and stably refluxed for 8-10 h and cooled to room temperature, the solvent is concentrated to 40-50%, the mixture is sealed and stays in an ice bath at 0 ∼ -5 °C for 10-20 hours, leading to precipitation of large amount of white solid, which is suction filtered, and the crude product is column chromatographed (eluted with 1:1 of petroleum ether:ethyl acetate) and vacuum dried to give the photochromic alkyl-naphthalene ring spiropyran compound (II); the specific reaction formula is as follow:

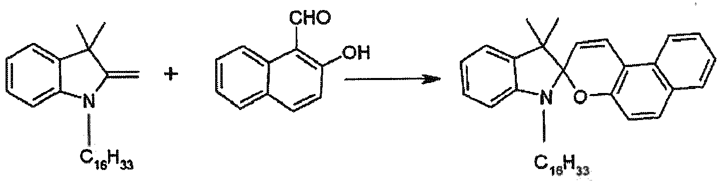

### (3) Synthesis of the photochromic spiropyran compound (III):

Step 1: 4-Hydroxy-6,7-dimethoxy-1-phenyl-2-naphthoic acid, o-xylene, and polyphosphoric acid with a mass ratio of (0.8-1.2):(1.5-2.5):(0.8-1.2) are heated to 120-160 °C, the mixture is reacted for 1-2 h, the reaction solution is poured into water, and the mixture is filtered and vacuum dried to give 5-hydroxy-2,3-dimethoxy-7H-benzo[c]fluorene-7-one;

Step 2: Tetrahydrofuran and 5-hydroxy-2,3-dimethoxy-7H-benzo[c]fluorene-7-one are mixed with a mass ratio of (0.6-1):1, the mixture is cooled to -10 ∼ -20 °C, methylmagnesium chloride (3.0 M) is dropwise added, the mass ratio of methylmagnesium chloride to 5-hydroxy-2,3-dimethoxy-7H-benzo[c]fluorene-7-one is (0.6-1.2):(0.7-1.2), and during the dropwise addition the temperature is controlled to be not higher than -10 °C. After completion of the dropwise addition, the temperature is kept at -10 ∼ -15 °C and the mixture is reacted for 90-120 minutes, a saturated ammonium chloride solution is added to quench the reaction, the mass ratio of ammonium chloride to methylmagnesium chloride is (1-3):1, the liquid is separated, the aqueous layer is extracted with ethyl acetate, the organic layers are combined and dried over anhydrous Na₂SO₄, the solvent is evaporated at reduced pressure, and the crude product is column chromatographed to give 2,3-dimethoxy-7-methyl-7H-benzo[c]fluorene-5,7-diol;

Step 3: 2,3-Dimethoxy-7-methyl-7H-benzo[c]fluorene-5,7-diol, toluene, 1,1-di(4-methoxyphenyl)-2-propyne-1-ol, and p-toluenesulfonic acid (TsOH) are uniformly mixed with a mass ratio of (0.7-1.0):(4-6):(0.6-1.0):(0.1-0.3), the mixture is heated to 65-95 °C and reacted for 10-16 h, toluene is evaporated at reduced pressure, a saturated sodium bicarbonate solution is added to conduct the quenching reaction, the mass ratio of the sodium bicarbonate solution to p-toluene is (0.3-0.6):1, the liquid is separated, the aqueous layer is extracted with ethyl acetate, the organic layers are combined and dried over anhydrous Na₂SO₄, the solvent is evaporated at reduced pressure, and the crude product is column chromatographed (eluted with petroleum ether:ethyl acetate = 15:1) to give the photochromic spiropyran compound (III).

The main reaction process is: 4-hydroxy-6,7-dimethoxy-1-phenyl-2-naphthoic acid is cyclized at an acidic condition to give the intermediate 5-hydroxy-2.3-dimethoxy-7H-benzo[c]fluorene-7-one, reacted with the Grignard reagent of methyl magnesium chloride to give 2,3-dimethoxy-7-methyl-7H-benzo[c]fluorene-5,7-diol, and then reacted with 1,1-di(4-methoxyphenyl)-2-propyne-1-ol under the catalytic action of p-toluenesulfonic acid to generate the photochromic spiropyran compound (III); the specific reaction formula is as follow:

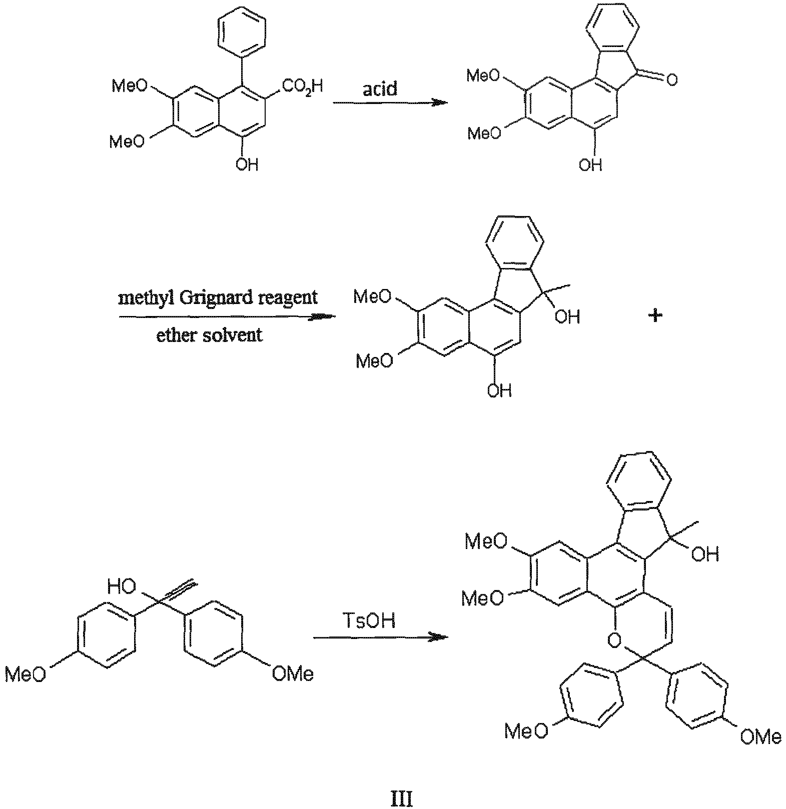

In the reaction the acid is acetic acid, trifluoroacetic acid, phosphoric acid, sulfuric acid or polyphosphoric acid, preferably polyphosphoric acid.

The photochromic nano-composite microsphere of the invention is a polyurethane/spiropyran/zinc sulfide three-layer composite core-shell structure, and its core is a zinc sulfide mesoporous nano-microsphere; in the preferred embodiment of the invention, the preparation route of the zinc sulfide mesoporous nano-microsphere is: ethylene glycol is condensed at a high temperature condition to produce an acetaldehyde, which provides a hydrogen atom as the reductant to convert the S simple substance into S²⁻, and subsequently Zn²⁺ and S²⁻ released by zinc nitrate hexahydrate combine to form the ZnS crystal nuclei; the crystal nuclei continues to grow based on the diffusion mechanism, leading to the gradual aggregation of the initial particle of the reaction (i.e., the zinc sulfide nano-crystalline) and formation of the secondary particle (i.e., the zinc sulfide nanosphere); and the nucleation reaches certain stage, at which mercaptoethanol plays an important role during the reaction: on one hand, efficiently stops the agglomeration of large amount of particles, on the other hand, mercaptoethanol restricts the continual growth of the particle, along with the decrease of the ionic concentration, and the nucleation of the nano-crystalline gradually stops; the specific nucleation reaction formula is as follow:

HOCH₂CH₂OH → CH₃CHO+H₂O (1)

S+2CH₃CHO →CH₃CO-OCCH₃+S²⁻+2H⁺ (2)

Zn²⁺+ S²⁻ →ZnS (s) (3)

Thus prepared zinc sulfide nano-microsphere is formed during the nucleation of the zinc sulfide nano-crystalline, and is the monodispersed zinc sulfide mesoporous nanosphere composed of many small nano-crystallines; due to the larger specific surface area and the higher surface energy of such zinc sulfide nanosphere having the mesoporous structure, the adsorption force of the zinc sulfide nanosphere in the alcohol solvent is enhanced.

The photochromic spiropyran compound subsequently added is uniformly dispersed on the surface and in the pore of the zinc sulfide microsphere in the form of nanoparticle. Finally, polyurethane is used as the shell for coating to constitute the nano-composite core-shell structure.

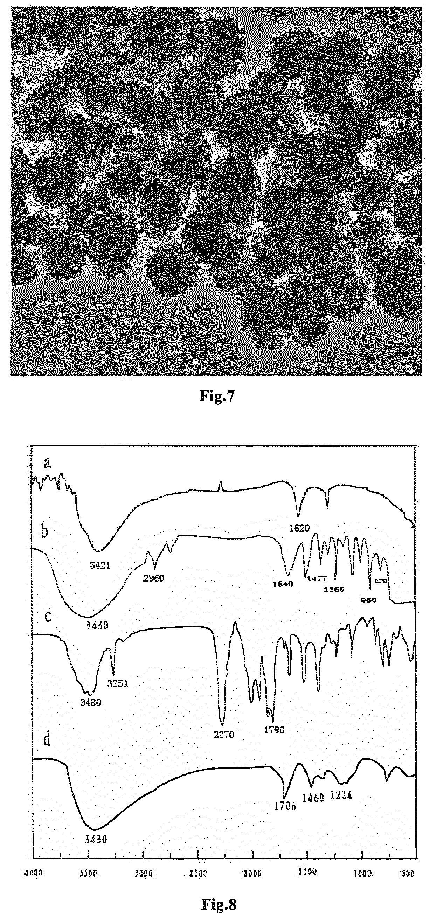

As shown in figure 7, the appearance of the photochromic nano-composite microsphere is spherical, wherein the core with a darker color is composed of many monodispersed zinc sulfide nanospheres of uniform size, and the outer layer with a light color is the photochromic spiropyran compound; because the polyurethane outer layer shell is a colorless clear material, it cannot be shown and resolved in the transmission electron microscope picture.

The beneficial effects of the invention are: the photochromic nano-composite microsphere described by the invention is a polyurethane/spiropyran/zinc sulfide three-layer composite core-shell structure, spiropyran is between the shell and the core as the photochromic compound (i.e., the middle layer), the spacial dimension is in the nanoscale range, the energy level brought by the quantum size effect changes, and the energy gap broadens. On one hand, this unique structure can cause the photochromic compound to be protected by the polyurethane shell having a relatively stable property, and protect the photochromic compound against the impact of the external environment; on the other hand, the stable supporting effect of the zinc sulfide nano-mesoporous structure of the core results in the increase of the Van der Waals area of the photochromic molecule, enlargement of the conjugated system, increase of the spacial volume of the molecule, and enlargement of the pore between the molecules, leading to great increase of the space for the isomerization reaction of the molecules, enhancement of the activity of the photochromic species, decrease of the hindrance of switching between the photochromic ring-open species and the colorless ring-closure species, shortening of the switching time, acceleration of the speed, increase of the sensitivity of the spectral response, and better photochromic effect. Furthermore, the polyurethane shell itself can absorb part of the light under the condition of light irradiation, part of the incoming UV light is absorbed by the zinc sulfide core, and the light intensity of the irradiation on the surface of the photochromic spiropyran compound is lower than the light intensity of the direct irradiation on the surface of the photochromic spiropyran compound; meanwhile, the irradiation intensity and the irradiation retention time needed to excite the spiropyran photochromism are not affected, and the remaining UV light will be absorbed by the zinc sulfide core. Therefore, the antioxidation property and the fatigue resistance of the polyurethane/spiropyran/zinc sulfide nano-composite microsphere are substantially enhanced.

The photochromic coating or the optical material prepared from the photochromic nano-composite microsphere can become colored from colorless under the UV light irradiation and rapidly fade to colorless when the UV light disappears, and have the advantages of strong fatigue resistance, good stability, and high photochromic speed, etc.

### Brief Description of the Drawings

Figure 1 is the normal environment absorption spectrum of the polyurethane/spiropyran/zinc sulfide nano-composite microsphere prepared in example 1.
Figure 2 is the high temperature environment absorption spectrum of the polyurethane/spiropyran/zinc sulfide nano-composite microsphere prepared in example 1.
Figure 3 is the normal environment absorption spectrum of the spiropyrans photochromic compound (III).
Figure 4 is the high temperature environment absorption spectrum of the spiropyrans photochromic compound (III).
Figure 5 is the normal environment absorption spectrum of the polyurethane/spiropyran nano-composite microsphere prepared in comparative example 2.
Figure 6 is the high temperature environment absorption spectrum of the polyurethane/spiropyran nano-composite microsphere prepared in comparative example 2.
Figure 7 is the transmission electron microscope picture of the photochromic nano-composite microsphere prepared in example 1.
Figure 8 is the infrared spectra of the photochromic nano-composite microsphere prepared in example 1, as well as the pure zinc sulfide, the photochromic spiropyran compound III, and polyurethane.

### Detailed Description of the Invention

The present invention will now be further illustrated by particular examples, but it is not intended for the claimed scope of the invention to be limited to the particular examples.

The spiropyrans photochromic compounds in the following examples and comparative examples were prepared using the following method:

### (1) Preparation of the photochromic spiropyran compound (I):

A. To 400 g of the chloroform solvent were added 29 g of 2,3,3-trimentylindole and 95 g of iodohexadecane, the mixture was heated and refluxed at atmospheric pressure,for 20 hours, after the solvent was evaporated, 70 g of anhydrous ethyl ether was added, after separation into layers, the mixture was suction filtered, sufficiently washed with ethyl ether, and vacuum dried to give hexadecane-2,3,3-trimentylindole (PS01); PS01 was dispersed in 170 g of water, a 26% NaOH solution was dropwise added with stirring, the dropwise addition was stopped after the solid completely disappeared, after separation into layers the mixture was extracted with ethyl ether, and the extract was washed with water, filtered, and rotavaped to remove ethyl ether to give 3,3-dimethyl-1'-hexadecyl-2-methylene indole (PS02);
B. Under protection of nitrogen an ethanol solution of 575 g of 4-hydroxy-benzene-1,3-dialdehyde (the solution contained 25 g of ethanol) was heated to reflux in an oil bath, heated to the temperature of 65 °C, 63 g of an ethanol solution containing PS02 (the solution contained 18 g of ethanol) was dropwise added, the color turned purple, the mixture was continually heated and refluxed for 6 hours and cooled to room temperature, the reaction solution was poured into an ice and stirred, after the ice completely melted, a light purple emulsion was obtained, after it sufficiently stayed, water and ethanol were evaporated, and the sticky material was recrystallized with methanol, suction filtered and dried to give a light purple solid powder, i.e., the photochromic alkyl spiropyran compound (I).

### (2) Preparation of the photochromic spiropyran compound (II):

Under protection of nitrogen 230 g of an ethanol solution containing 2-hydroxy-1-naphthaldehyde (the solution contained 10 g of ethanol) was heated to 60 °C and refluxed in an oil bath, 24 g of ethanol solution of PS02 (the solution contained 5 g of ethanol) was dropwise added, the mixture was continually refluxed for 9 hours, cooled to room temperature, concentrated to 40% by evaporation of the solvent, sealed and stayed in an ice bath at -2 °C for 15 hours and suction filtered, and the crude product was eluted with petroleum ether:ethyl acetate (1:1) and vacuum dried to give the photochromic alkyl-naphthalene ring spiropyran compound (II)

### (3)Preparation of the photochromic spiropyran compound (III):

Step 1: 90 g of 4-hydroxy-6,7-dimethoxy-1-phenyl-2-naphthoic acid, 170 g of o-xylene, and 85 g of polyphosphoric acid were stirred and mixed uniformly, heated to 145 °C, and reacted for 90 minutes, the reaction solution was poured into water, filtered, and vacuum dried to give 5-hydroxy-2,3-dimethoxy-7H-benzo[c]fluorene-7-one;

Step 2: 75 g of tetrahydrofuran and 95 g of 5-hydroxy-2,3-dimethoxy-7H-benzo[c]fluorene-7-one were mixed and cooled to -12 °C, 80 g of methylmagnesium chloride (3.0 M) was dropwise added, the mixture was reacted at -10 °C for 100 minutes, 150 g of a saturated ammonium chloride solution was added, the aqueous layer was extracted with ethyl acetate (170 mL × 3), the organic layers were combined and dried over anhydrous Na₂SO₄, the solvent was removed at reduced pressure, and the crude product was column chromatographed to give 2,3-dimethoxy-7-methyl- 7H-benzo[c]fluorene-5,7-diol;

Step 3: 40 g of 2,3-dimethoxy-7-methyl-7H-benzo[c]fluorene-5,7-diol, 250 g of toluene, 42 g of 1,1-di(4-methoxyphenyl)-2-propyne-1-ol, and 9 g of p-toluenesulfonic acid were stirred and mixed uniformly, the mixture was heated to 75 °C and reacted for 15 hours, toluene was evaporated at reduced pressure, 110 g of a saturated sodium bicarbonate solution was added to quench the reaction, the aqueous layer was extracted with ethyl acetate (90 mL × 3), the organic layers were combined and dried over anhydrous Na₂SO₄, the solvent was evaporated at reduced pressure, and the crude product was column chromatographed to give the photochromic spiropyran compound (III).

### Example 1: Preparation of the photochromic nano-composite microsphere, the photochromic coating liquid and the photochromic lens

### [1] Preparation of the photochromic nano-composite microsphere:

(1) To 1600 g of ethylene glycol was added 9.7 g of zinc nitrate hexahydrate, the mixture was uniformly stirred, 1.6 g of sublimed sulfur powder was added, the temperature was elevated to 150 °C, the mixture was reacted at constant temperature for 24 hours and cooled to 55 °C, 85 g of mercaptoethanol was dropwise added, and the mixture was stirred; 6.7 g of the photochromic spiropyran compound shown in formula III was added, the mixture was vigorously stirred for 30 minutes and cooled to room temperature, and the precipitate was filtered, washed, and dried in a vacuum drying oven at 65 °C for 8 hours to give the spiropyrans-coated zinc sulfide nanosphere;
(2) To 1000 g of the butyl acetate solvent were added 8 g of the spiropyrans-coated zinc sulfide nanosphere and 8.2 g of diphenylmethane-4,4'-diisocyanate (MDI), the mixture was uniformly stirred, 7 g of mixed reactants (1.2 g of n-butanol, 5.8 g of polytetramethylene ether glycol) and 18 g of Tween 80 were added respectively, the temperature was kept at 20 °C, the mixture was prepolymerized for 60 minutes at a rotational speed of 500 r/min, 9.2 g of chain extender (6 g of ethylene glycol, 3.2 g of ethylene diamine) and 0.1 g of catalyst of dibutyltin dilaurate (DBTL) were added, the mixture was heated to 60 °C to conduct the chain extending polymerization reaction for 15 minutes at a stirring speed of 1200 r/min, and the solid material was filtered, washed, and dried to give polyurethane/spiropyran/zinc sulfide nano-composite microsphere. The yield was 75%.
(3) The product prepared in step (2) was visualized with Type JEM-2100 transmission electron microscope; as shown in figure 7, it can be seen that the appearance was spherical, wherein the core with a darker color was composed of many monodispersed zinc sulfide nanospheres of uniform size, the outer layer with a light color was a shell formed from the photochromic spiropyran compound and polyurethane, they can be attributed to the core-shell structure composite microsphere formed by the zinc sulfide nanosphere having a larger specific surface area and pore volume adsorbing the photochromic spiropyran compound material, followed by coating of polyurethane, and because the polyurethane outer layer shell was a colorless clear material, it cannot be shown and resolved in the transmission electron microscope picture. It was worth noting that there are lots of nano-sized white spots in some nanospheres, demonstrating that the nanosphere was formed by aggregation of lots of small particles; the white spot was the pore volume formed by the pores between particles, and the average crystal size of zinc sulfide and the diameter of the nano-composite microsphere are inferred by the calculation through the Scherrer Equation (D = K/βcos θ) and the Zeta-Potential analysis, wherein the diameter of the zinc sulfide nano-crystalline was 4 nm, the thickness of the shell coated by the photochromic spiropyran material and polyurethane was around 15 nm, the zinc sulfide nanosphere core was around 65 nm, and the diameter of the whole composite microsphere was around 95nm.
(4) A fourier transform infrared spectroscopy (FT-IR) test was carried out for the product prepared in step (2), and the spectra are compared with the infrared spectra of pure zinc sulfide, photochromic spiropyran compound III, and polyurethane. See figure 8 for detailed results, wherein curve a is the infrared spectrum of zinc sulfide; curve b is the infrared spectrum of the spiropyran compound III; curve c is the infrared spectrum of the polyurethane material; curve d is the infrared spectrum of the photochromic nano-composite microsphere prepared in example 1.

It can be known from the analysis of curve a in the figure that the broader absorption peak at 3421 cm⁻¹ is the peak for the stretching vibration of O-H, the absorption peak at 1620 cm⁻¹ is the peak for the bending vibration of H-O-H, both are characteristic absorption peaks of water, and can be attributed to the water adsorbed on the surface of the ZnS powder; there is substantially no absorption peak in the range of 900-4000 cm⁻¹, demonstrating that the ZnS powder has good degree of infrared transparency.

It can be known from the analysis of curve b in the figure that the absorption peak at 3430 cm⁻¹ is for the stretching vibration of -OH, the absorption peak at 2960 cm⁻¹ is for the stretching vibration of -CH₃, the absorption peaks at 1640-1477 cm⁻¹ are attributed to the vibration of the benzene ring, the absorption peak at 1366 cm⁻¹ is for the bending vibration of -CH₃, the absorption peak at 960 cm⁻¹ is for the stretching vibration of -CH₃, and the absorption peak at 820 cm⁻¹ is for the vibration of the ortho-substituents of the benzene ring. It can be known from the analysis of curve c in the figure that the absorption peaks at 3251-3480 are the peaks of stretching vibration of the hydroxy-OH of polyurethane; the strong absorption peak at 2270 cm⁻¹ is generated by the stretching vibration of the -NCO group, the other groups do not have absorption at the position of this peak, and this peak is the characteristic absorption peak of isocyanate.

It can be known from the analysis of curve d in the figure that the absorption peak for the stretching vibration of -OH is at 3430 cm⁻¹, the vibration absorption peak disappears at 2270 cm⁻¹, indicating that the -NCO group does not exist because the MDI monomer is completed reacted, isocyanate has reacted with the compound having weak active hydrogen atoms to generate urethane, the absorption peak at 1706 cm⁻¹ represents the vibration absorption peak of -C=O of polyurethane; the absorption peak at 1460 cm⁻¹ is the vibration absorption peak of the CH₂ group (methylene) or the vibration absorption peak of CH₃ (methyl), though not consistent with the absorption wavenumber of the spiropyran molecule (1366 cm⁻¹), probably because of the interaction between different molecules after the spiropyran molecule is adsorbed onto the ZnS nanoparticle, shift of the absorption spectra occurs; the absorption peak at 1224 cm⁻¹ is the vibration absorption peak of the ether bond -C-O-C-.

It can be seen from the comparison that, the infrared spectra of the photochromic nano-composite microsphere prepared in example 1 not only contains the characteristic absorption peak of the photochromic spiropyran compound, but also contains the characteristic absorption peak of polyurethane, indicating that the photochromic nano-composite microsphere prepared in example 1 is not the simple zinc sulfide material, and contains the photochromic spiropyran compound and the polyurethane material.

### [2] Preparation of the photochromic coating liquid:

1. Formulating the polyurethane Component A: 675 g of 2,3-dimercaptoethylthiopropanethiol (BES) monomer, 120 g of dichloromethane, 35 g of the polyurethane/spiropyran/zinc sulfide nano-composite microsphere, 15 g of the F420 wetting dispersing agent (a product of Foshan Aona Polymer Co. Ltd.), and 5 g of the F300 leveling agent (a product of Foshan Aona Polymer Co. Ltd.) were added into a container, and the mixture was uniformly stirred at room temperature for 25 minutes to give Component A;
2. Formulating the polyurethane Component B: 145 g of the m-xylylene diisocyanate curing agent, 4 g of the XPJ01F defoamer (a product of Jiangsu Saiouxinyue Defoamer Agent Co., Ltd.), and 1 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 600 g of the mixture as the first photochromic coating liquid; Container B contained 400 g of the mixture, and 26 g of dichloromethane was added into Container B to give the second photochromic coating liquid.

### [3] Preparation of the photochromic lens:

The substrate of the polyurethane optical lens after treatment by cleaning with NaOH was immerged into the first photochromic coating liquid described above and soaked at room temperature for 3 minutes, the lens was then slowly pulled, the pulling speed was 1.0 mm/s, after 85 s the speed was increased to 1.3 mm/s, and the lens was moved to an oven set at a temperature of 45 °C and baked for 80 minutes; the lens was spray-coated with the second photochromic liquid and cured to give the photochromic spiropyran lens, wherein the thickness of the coating was 45 µm.

### Example 2

### [1] Preparation of the photochromic nano-composite microsphere:

(1) To 1600 g of ethylene glycol was added 6.5 g of zinc nitrate, the mixture was uniformly stirred, 1.7 g of sublimed sulfur powder was added, the temperature was elevated to 150 °C, the mixture was reacted at constant temperature for 24 hours and cooled to 55 °C, 70 g of mercaptoethanol was dropwise added, and the mixture was stirred; 3.5 g of the photochromic spiropyran compound (I) was added, the mixture was vigorously stirred for 30 minutes and cooled to room temperature, and the precipitate was filtered, washed, and dried in a vacuum drying oven at 65 °C for 8 hours to give the spiropyrans-coated zinc sulfide nanosphere;
(2) To 1000 g of the butyl acetate solvent were added 5.5 g of the spiropyrans-coated zinc sulfide nanosphere and 5 g of MDI, the mixture was uniformly stirred, 5.5 g of mixed reactants (1.0 g of n-butanol, 4.5 g of polytetramethylene ether glycol) and 20 g of Tween 80 were sequentially added, the temperature was kept at 25 °C, the mixture was prepolymerized at a rotational speed of 500 r/min for 50 minutes, 6 g of chain extender (4.5 g of ethylene glycol, 1.5 g of ethylene diamine) and 0.1 g of DBTL were added, the mixture was heated to 65 °C to conduct the chain extending polymerization reaction for 15 minutes at a stirring speed of 1000 r/min, and the solid material was filtered, washed, and dried to give the polyurethane/spiropyran/zinc sulfide nano-composite microsphere. The yield was 74%.
   The Type JEM-2100 transmission electron microscope picture of the product was similar to figure 7.

### [2] Preparation of the photochromic coating liquid:

1. Formulating the polyurethane Component A: 610 g of the 1,2,3-trimercapto-ethylthiopropane (TES) monomer, 100 g of dichloromethane, 40 g of the polyurethane/spiropyran/zinc sulfide nano-composite microsphere, 10 g of the F420 wetting dispersing agent, and 5 g of the F320 leveling agent (a product of Foshan Aona Polymer Co. Ltd.) were added into a container, and the mixture was uniformly stirred at room temperature for 20 minutes to give Component A;
2. Formulating the polyurethane Component B: 150 g of the m-xylylene diisocyanate curing agent, 6 g of the XPJ01F defoamer, and 3 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 600 g of the mixture as the first photochromic coating liquid; Container B contained 324 g of the mixture, and 20 g of dichloromethane was added into Container B to give the second photochromic coating liquid.

### [3] Preparation of the photochromic lens:

The substrate of the polyurethane optical lens after the hardening treatment was immerged into the first photochromic coating liquid described above and soaked at room temperature for 3 minutes, the lens was then slowly pulled, the pulling speed was 1.0 mm/s, after 85 s the speed was increased to 1.3 mm/s, and the lens was moved to an oven set at a temperature of 45 °C and baked for 70 minutes; the lens was spray-coated with the second photochromic liquid and cured to give the photochromic spiropyran lens, wherein the thickness of the coating was 43 µm.

### Example 3

### [1] Preparation of the photochromic nano-composite microsphere:

(1) To 1600 g of ethylene glycol was added 11 g of zinc nitrate hexahydrate, the mixture was uniformly stirred, 3 g of sulfur powder was added, the temperature was elevated to 145 °C, the mixture was reacted at constant temperature for 24 hours and cooled to 50 °C, 92 g of mercaptoethanol was dropwise added, and the mixture was stirred; 8 g of the photochromic spiropyran compound (I) was added, the mixture was vigorously stirred for 35 minutes and cooled to room temperature, and the precipitate was filtered, washed, and dried in a vacuum drying oven at 60 °C for 9 hours to give the spiropyrans-coated zinc sulfide nanosphere;
(2) To 1000 g of the butyl acetate solvent were added 10 g of the spiropyrans-coated zinc sulfide nanosphere and 10.8 g of m-xylylene diisocyanate (XDI), the mixture was uniformly stirred, 7.4 g of mixed reactants (1.0 g of n-butanol, 6.4 g of polytetramethylene ether glycol) and 16 g of Tween 80 were sequentially added, the temperature was kept at 25 °C, the mixture was prepolymerized at a rotational speed of 600 r/min for 60 minutes, 8 g of chain extender (5.5 g of ethylene glycol, 2.5 g of ethylene diamine) and 0.2 g of DBTL were added, the mixture was heated to 60 °C to conduct the chain extending polymerization reaction for 15 minutes at a stirring speed of 1300 r/min, and the solid material was filtered, washed, and dried to give the polyurethane/spiropyran/zinc sulfide nano-composite microsphere. The yield was 73%.
   The Type JEM-2100 transmission electron microscope picture of the product was similar to figure 7.

### [2] Preparation of the photochromic coating liquid:

1. Formulating the polyurethane Component A: 710 g of the TES monomer, 130 g of dichloromethane, 50 g of the polyurethane/spiropyran/zinc sulfide nano-composite microsphere, 18 g of the F420 wetting dispersing agent, and 10 g of the F320 leveling agent were added into a container, and the mixture was uniformly stirred at room temperature for 25 minutes to give Component A;
2. Formulating the polyurethane Component B: 140 g of the m-toluene diisocyanate curing agent, 6 g of the XPJ01F defoamer, and 5 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 600 g of the mixture as the first photochromic coating liquid; Container B contained 469 g of the mixture, and 30 g of dichloromethane was added into Container B to give the second photochromic coating liquid.

### [3] Preparation of the photochromic lens:

The substrate of the polyurethane optical lens after the acid wash treatment was immerged into the first photochromic coating liquid described above and soaked at room temperature for 3 minutes, the lens was then slowly pulled, the pulling speed was 1.0 mm/s, after 90 s the speed was increased to 1.3 mm/s, and the lens was moved to an oven set at a temperature of 45 °C and baked for 70 minutes; with the same method as the first dip-coating, the lens was again immerged into the second photochromic coating liquid and cured to give the photochromic spiropyran lens, wherein the thickness of the coating was 45 µm.

### Example 4

### [1] Preparation of the photochromic nano-composite microsphere:

(1) To 1600 g of ethylene glycol was added 14 g of zinc nitrate hexahydrate, the mixture was uniformly stirred, 2 g of sublimed sulfur powder was added, the temperature was elevated to 150 °C, the mixture was reacted at constant temperature for 23 hours and cooled to 50 °C, 100 g of mercaptoethanol was dropwise added, and the mixture was stirred; 12 g of the photochromic spiropyran compound (II) was added, the mixture was vigorously stirred for 35 minutes and cooled to room temperature, and the precipitate was filtered, washed, and dried in a vacuum drying oven at 60 °C for 9 hours to give the spiropyrans-coated zinc sulfide nanosphere;
(2) To 1000 g of the butyl acetate solvent were added 12 g of the spiropyrans-coated zinc sulfide nanosphere and 11.5 g of XDI, the mixture was uniformly stirred, 8.5 g of mixed reactants (1 g of n-butanol, 7.5 g of polytetramethylene ether glycol) and 20 g of Tween 80 were sequentially added, the temperature was kept at 20 °C, the mixture was prepolymerized for 60 minutes at a rotational speed of 650 r/min, 8 g of chain extender (5.5 g of ethylene glycol, 2.5 g of ethylene diamine) and 0.2 g of DBTL were added, the mixture was heated to 60 °C to conduct the chain extending polymerization reaction for 15 minutes at a stirring speed of 1200 r/min, and the solid material was filtered, washed, and dried to give the polyurethane/spiropyran/zinc sulfide nano-composite microsphere. The yield was 72%.
   The Type JEM-2100 transmission electron microscope picture of the product was similar to figure 7.

### [2] Preparation of the photochromic coating liquid:

1. Formulating the polyurethane Component A: 750 g of the 2,2'-dimercaptodiethylsulfide (MES) monomer, 150 g of dichloromethane, 50 g of the polyurethane/spiropyran/zinc sulfide nano-composite microsphere, 20 g of the F420 wetting dispersing agent, and 10 g of the F300 leveling agent were added into a container, and the mixture was uniformly stirred at room temperature for 25 minutes to give Component A;
2. Formulating the polyurethane Component B: 120 g of the methylcyclohexyl diisocyanate curing agent, 7 g of the XPJ01F defoamer, and 6 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 600 g of the mixture as the first photochromic coating liquid; Container B contained 513 g of the mixture, and 30 g of dichloromethane was added into Container B to give the second photochromic coating liquid.

### [3] Preparation of the photochromic lens:

The substrate of the polyurethane optical lens after the hardening treatment was immerged into the first photochromic coating liquid described above and soaked at room temperature for 3 minutes, the lens was then slowly pulled, the pulling speed was 0.9 mm/s, after 95 s the speed was increased to 1.4 mm/s, and the lens was moved to an oven set at a temperature of 50 °C and baked for 75 minutes; with the same method as the first dip-coating, the lens was again immerged into the second photochromic coating liquid and cured to give the photochromic spiropyran lens, wherein the thickness of the coating was 44 µm.

### Example 5

### [1] Preparation of the photochromic nano-composite microsphere:

(1) To 1600 g of ethylene glycol was added 8 g of zinc nitrate hexahydrate, the mixture was uniformly stirred, 2.5 g of sublimed sulfur powder was added, the temperature was elevated to 150 °C, the mixture was reacted at constant temperature for 23 hours and cooled to 50 °C, 82 g of mercaptoethanol was dropwise added, and the mixture was stirred; 5.2 g of the photochromic spiropyran compound (III) was added, the mixture was vigorously stirred for 35 minutes and cooled to room temperature, and the precipitate was filtered, washed, and dried in a vacuum drying oven at 60 °C for 9 hours to give the spiropyrans-coated zinc sulfide nanosphere;
(2) To 1000 g of the butyl acetate solvent were added 6.6 g of the spiropyrans-coated zinc sulfide nanosphere and 5.6 g of toluene diisocyanate (TDI), the mixture was uniformly stirred, 6.3 g of mixed reactants (0.9 g of n-butanol, 5.4 g of polytetramethylene ether glycol) and 14 g of Tween 80 were sequentially added, the temperature was kept at 25 °C, the mixture was prepolymerized at a rotational speed of 700 r/min for 60 minutes, 5.5 g of chain extender (4.5 g of ethylene glycol, 1 g of ethylene diamine) and 0.1 g of DBTL were added, the mixture was heated to 60 °C to conduct the chain extending polymerization reaction for 15 minutes at a stirring speed of 1500 r/min, and the solid material was filtered, washed, and dried to give the polyurethane/spiropyran/zinc sulfide nano-composite microsphere. The yield was 74%.
   The Type JEM-2100 transmission electron microscope picture of the product was similar to figure 7.

### [2] Preparation of the photochromic coating liquid:

1. Formulating the polyurethane Component A: 640 g of the BES monomer, 110 g of dichloromethane, 25 g of the polyurethane/spiropyran/zinc sulfide nano-composite microsphere, 17 g of the F420 wetting dispersing agent, and 5 g of the F309 leveling agent were added into a container, and the mixture was uniformly stirred at room temperature for 25 minutes to give Component A;
2. Formulating the polyurethane Component B: 110 g of the m-xylylene diisocyanate curing agent, 9 g of the XPJ01F defoamer, and 7 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 700 g of the mixture as the first photochromic coating liquid; Container B contained 223 g of the mixture, and 15 g of dichloromethane was added into Container B to give the second photochromic coating liquid.

### [3] Preparation of the photochromic lens:

The substrate of the polyurethane optical lens after the hardening treatment was immerged into the first photochromic coating liquid described above and soaked at room temperature for 3 minutes, the lens was then slowly pulled, the pulling speed was 1.1 mm/s, after 85 s the speed was increased to 1.34 mm/s, and the lens was moved to an oven set at a temperature of 45 °C and baked for 70 minutes; the lens was spray-coated with the second photochromic liquid and cured to give the photochromic spiropyran lens, wherein the thickness of the coating was 43 µm.

### Example 6

### [1] Preparation of the photochromic nano-composite microsphere:

(1) To 1600 g of ethylene glycol was added 8 g of zinc nitrate hexahydrate, the mixture was uniformly stirred, 2.7 g of sublimed sulfur powder was added, the temperature was elevated to 150 °C, the mixture was reacted at constant temperature for 23 hours and cooled to 45 °C, 80 g of mercaptoethanol was dropwise added, and the mixture was stirred; 5.9 g of the photochromic spiropyran compound (II) was added, the mixture was vigorously stirred for 35 minutes and cooled to room temperature, and the precipitate was filtered, washed, and dried in a vacuum drying oven at 60 °C for 9 hours to give the spiropyrans-coated zinc sulfide nanosphere;
(2) To 1000 g of the butyl acetate solvent were added 7.2 g of the spiropyrans-coated zinc sulfide nanosphere and 6.9 g of TDI, the mixture was uniformly stirred, 6.5 g of mixed reactants (1.5 g of n-butanol, 5.0 g of polytetramethylene ether glycol) and 12 g of Tween 80 were sequentially added, the temperature was kept at 25 °C, the mixture was prepolymerized at a rotational speed of 500 r/min for 60 minutes, 7 g of chain extender (5.5 g of ethylene glycol, 1.5 g of ethylene diamine) and 0.1 g of DBTL were added, the mixture was heated to 70 °C to conduct the chain extending polymerization reaction for 15 minutes at a stirring speed of 1500 r/min, and the solid material was filtered, washed, and dried to give the polyurethane/spiropyran/zinc sulfide nano-composite microsphere. The yield was 73%.
   The Type JEM-2100 transmission electron microscope picture of the product was similar to figure 7.

### [2] Preparation of the photochromic coating liquid:

1. Formulating the polyurethane Component A: 660 g of the MES monomer, 140 g of dichloromethane, 30 g of the polyurethane/spiropyran/zinc sulfide nano-composite microsphere, 16 g of the F420 wetting dispersing agent, and 5 g of the F320 leveling agent were added into a container, and the mixture was uniformly stirred at room temperature for 25 minutes to give Component A;
2. Formulating the polyurethane Component B: 100 g of the diphenylmethane-4,4'-diisocyanate curing agent, 8 g of the XPJ01F defoamer, and 8 g of the DBTL catalyst were added into a container, and the mixture was uniformly stirred at room temperature for 5 minutes to give Component B;
3. Formulating the photochromic coating liquid: Component A and Component B were uniformly mixed and separately charged into Container A and B, wherein Container A contained 600 g of the mixture as the first photochromic coating liquid; Container B contained 367 g of the mixture, and 17 g of dichloromethane was added into Container B to give the second photochromic coating liquid.

### [3] Preparation of the photochromic lens:

The substrate of the polyurethane optical lens after the hardening treatment was immerged into the first photochromic coating liquid described above and soaked at room temperature for 4 minutes, the lens was then slowly pulled, the pulling speed was 0.9 mm/s, after 80 s the speed was increased to 1.4 mm/s, and the lens was moved to an oven set at a temperature of 60 °C and baked for 60 minutes; with the same method as the first dip-coating, the lens was again immerged into the second photochromic coating liquid and cured to give the photochromic spiropyran lens, wherein the thickness of the coating was 45 µm.

### Comparative example 1

The photochromic coating liquid and the photochromic lens were prepared using the same operation condition as steps [2] and [3] of example 1, except that the photochromic spiropyran compound (III) was used to replace the polyurethane/spiropyran/zinc sulfide nano-composite microsphere during the preparation of the photochromic coating liquid.

### Comparative example 2

(1) The same operation condition as step [1] - (2) of example 1 was used, except that the photochromic spiropyran compound (III) was used to replace the spiropyrans-coated zinc sulfide nanosphere, to give the polyurethane/spiropyran nano-composite microsphere.
(2) The photochromic coating liquid and the photochromic lens were prepared using the same operation condition as steps [2] and [3] of example 1, except that the polyurethane/spiropyran nano-composite microsphere obtained in step (1) described above was used to replace the polyurethane/spiropyran/zinc sulfide nano-composite microsphere during the preparation of the photochromic coating liquid.

### Example 7

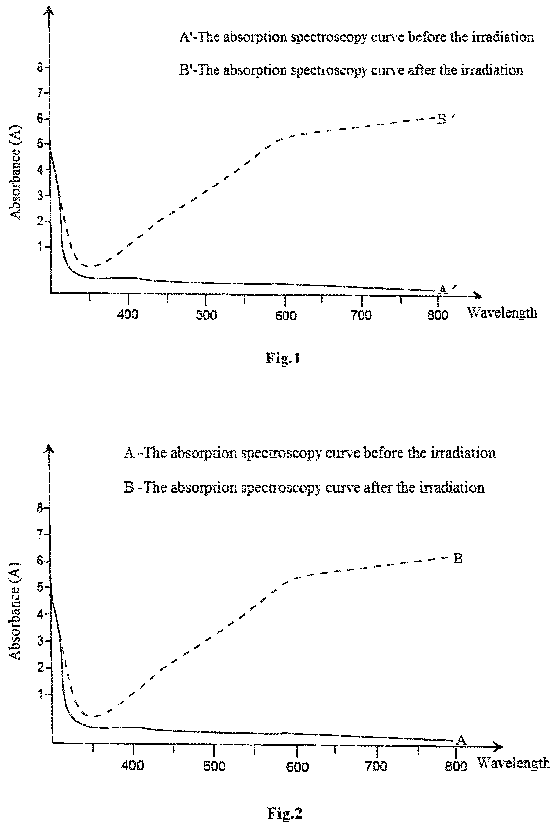

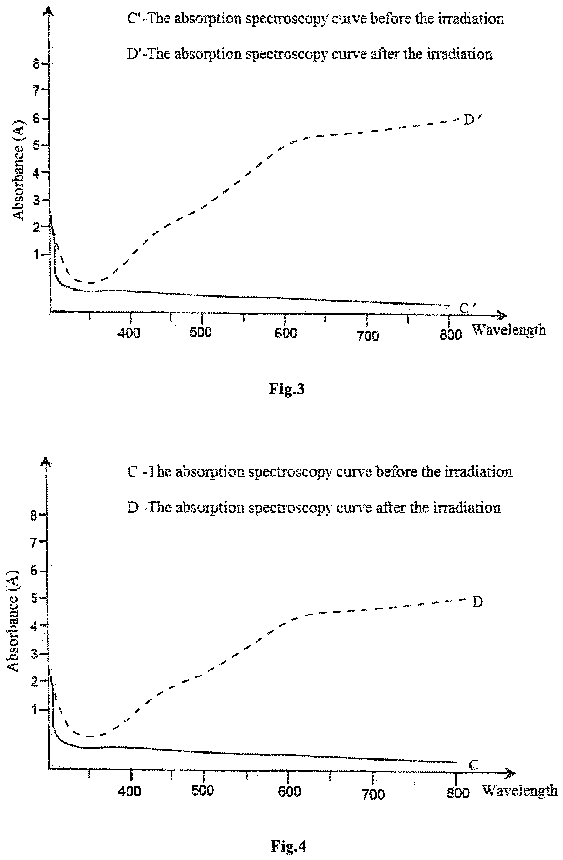

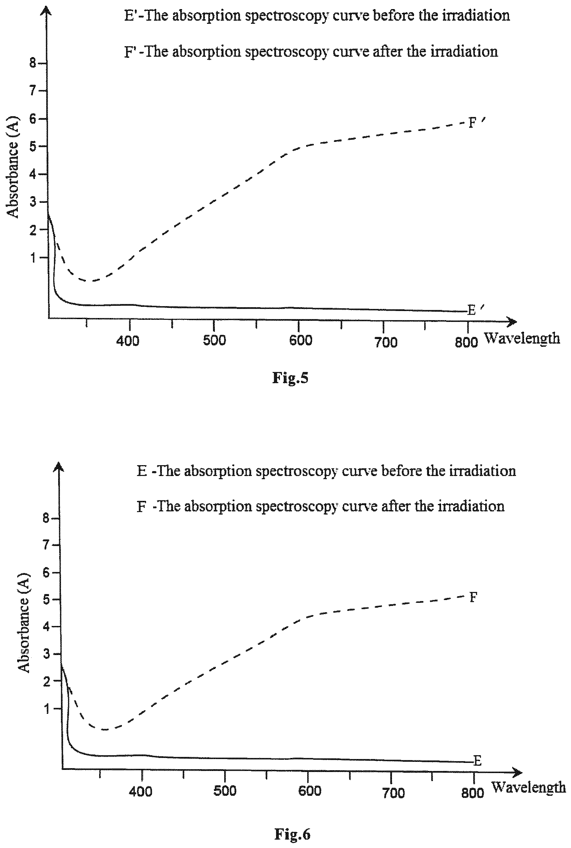

The antioxidation property test was performed for the photochromic material prepared in example 1 and comparative examples 1-2, respectively.
[1] The absorption spectroscopy test was conducted to the polyurethane/spiropyran/zinc sulfide nano-composite microsphere and the spiropyrans photochromic compound (III) prepared in example 1 and the polyurethane/spiropyran nano-composite microsphere prepared in comparative example 2, respectively;
(1) Testing procedure: 5 gram of each of the materials described above was dissolved in 9 mL tetrahydrofuran, the mixture was separately charged into several glass beakers, after the beakers were labeled, one part of each was separately put into the sun light simulation box to conduct the irradiation test, irradiated at normal temperature for 15 minutes, the distance between the solution and the light source was 16 CM, the radiation quantity hv = 2 Eg, the absorption spectra difference of the solution before and after the irradiation was recorded, and the test results were as shown in figure 1, figure 3, figure 5 and table 1.

**Table 1. Absortion Spectra Difference of the Solution at Normal Environment (ΔOD)**

| Absorbance Test Sample | Absorption Spectra Difference of the Solution Before and After the Irradiation | |
|---|---|---|
| | 550 nm | 750 nm |
| Example 1 | 3.9 | 5.9 |
| Comparative Example 1 | 3.9 | 5.9 |
| Comparative Example 2 | 3.9 | 5.9 |

(2) Testing procedure: 5 g of the polyurethane/spiropyran/zinc sulfide nano-composite microsphere and 5 g of the spiropyrans photochromic compound (III) prepared in example 1 and 5 g of the polyurethane/spiropyran nano-composite microsphere prepared in comparative example 2 were separately put into 3 crucibles, and the crucibles were moved into a muffle furnace, all heated to 200 °C, and stayed at this temperature for 28 hours; the photochromic compound described above was taken out and separately dissolved in 9 mL tetrahydrofuran, the mixture was separately charged into several glass beakers, after the beakers were labeled, one part of each was separately put into the sun light simulation box to conduct the irradiation test, and irradiated at normal temperature for 15 minutes, the distance between the solution and the light source was 16 CM, the radiation quantity hv = 2 Eg, the absorption spectra difference of the solution before and after the irradiation was recorded, and the test results were as shown in figure 2, figure 4, figure 6 and table 2.

**Table 2. Absorption Spectra Difference of the Solution at High Temperature Environment (ΔOD)**

| Absorbance Test Sample | Absorption Spectra Difference of the Solution Before and After the Irradiation | |
|---|---|---|
| | 550 nm | 750 nm |
| Example 1 | 3.9 | 5.9 |
| Comparative Example 1 | 3.0 | 4.9 |
| Comparative Example 2 | 3.0 | 4.9 |

[2] The ageing test was conducted to the photochromic lens prepared in examples 1-6 and comparative examples 1-2, respectively.

Testing procedure and method: the irradiation ageing resistance test was conducted to the photochromic lens prepared in examples 1-6 and comparative examples 1-2, respectively; Type ZN-P UV light ageing testing box of Wuxi Zhongtian Engineering Technology Co., Ltd. was selected as the test equipment, the temperature was set at 60 °C, the irradiation limit was selected as the irradiation intensity; the photochromic lens was labeled and irradiated for 15 minutes and 48 hours, respectively, and the transmission ratio data before and after the irradiation were recorded; see table 3 for the test results.

**Table 3. Transmission Ratio of the Photochromic Lens (%)**

| Transmission Ratio Test Sample | Irradiation for 15 Minutes Transmission Ratio at 600 nm | Irradiation for 48 Hours Transmission Ratio at600 nm | Relative Change |
|---|---|---|---|
| Example 1 | 48 | 48 | 0 |
| Example 2 | 50 | 50 | 0 |
| Example 3 | 50 | 50 | 0 |
| Example 4 | 51 | 51 | 0 |
| Example 5 | 48 | 48 | 0 |
| Example 6 | 51 | 51 | 0 |
| Comparative Example 1 | 49 | 64 | 15 |
| Comparative Example 2 | 49 | 65 | 16 |
| Note: Relative changes less than 1% were omitted and not recorded. | | | |

Testing conclusion: [1] the absorption spectra of the solution containing the polyurethane/spiropyran/zinc sulfide nano-composite microsphere didn't change at the normal environment and the high temperature environment (no difference), whereas the absorption spectra of the photochromic liquid prepared in comparative examples 1-2 at the normal environment and the high temperature environment changed, the change value at 550 nm reached 0.9, and the change value at 750 nm reached 1; [2] There was no difference for the transmission ratio data of the photochromic lens containing the polyurethane/spiropyran/zinc sulfide nano-composite microsphere coating before and after the irradiation, whereas there was a relatively big difference for the transmission ratio data of the photochromic lens prepared in comparative examples 1-2 before and after the irradiation, and the difference reached 15 and 16, respectively.

The fatigue resistance of the photochromic material was mostly embodied by the absorbance or the change of the degree of transparency after the color change, therefore, the change of the absorbance and the change of the degree of transparency before and after the irradiation were used to evaluate the fatigue resistance, and by the comparison of the test data the following conclusion was drawn: the fatigue resistance of the optical material containing the polyurethane/spiropyran/zinc sulfide nano-composite microsphere was stronger.

### Example 8

The photochromic response test was carried out on the resin lens prepared in examples 1-6 and comparative examples 1-2, respectively.

### [1] The photochromic response value test

Testing procedure and method: the ratio of the light transmission ratio tV (0) of the testing sample at the fading state to the light transmission ratio tV (1) of the testing sample at the photochromic state after different light irradiation times (1-15 min), i.e., the spectral response value = tV (0) ÷ tV (1), was used to determine the photochromic response values of the testing samples at different temperatures. After the radiant intensity of the solar simulator dropped by 25 %, the sample was irradiated with moderate light intensity with the temperature set at 23 °C; see table 4 for the test results.

**Table 4. Photochromic Response Value of the Test Samples**

| Spectral Response Value Test Sample | Spectral Response Value (1 min) | Spectral Response Value (5 min) | Spectral Response Value (15 min) |
|---|---|---|---|
| Example 1 | 1.29 | 1.46 | 1.78 |
| Example 2 | 1.25 | 1.43 | 1.76 |
| Example 3 | 1.27 | 1.43 | 1.76 |
| Example 4 | 1.20 | 1.40 | 1.73 |
| Example 5 | 1.30 | 1.46 | 1.78 |
| Example 6 | 1.21 | 1.42 | 1.73 |
| Comparative Example 1 | 1.14 | 1.39 | 1.78 |
| Comparative Example 2 | 1.14 | 1.39 | 1.78 |

### [2] The photochromic spectral response time test

Testing procedure and method: the photochromic lens prepared in examples 1-6 and comparative examples 1-2 were put into a testing box, respectively, the light source of the sun light simulator was switched on, and the reaction time needed for the lens to become colored from colorless was recorded; the simulated light source was switched off after 10 minutes of irradiation, and the time needed for the lens to recover to the colorless state from colored was recorded; see table 5 for the test results.

**Table 5. Spectral Response Time of the Samples**

| Spectral Response Time Test Sample | UV light Irradiation Photochromic Reaction Time (S) | Fading Recovery Time After Being Moved to Darkness (S) | Color Change |
|---|---|---|---|
| Example 1 | 15 | 76 | colorless-green |
| Example 2 | 24 | 95 | colorless-purple |
| Example 3 | 25 | 94 | colorless-purple |
| Example 4 | 18 | 88 | colorless-pink |
| Example 5 | 15 | 75 | colorless-green |
| Example 6 | 19 | 87 | colorless-pink |
| Comparative Example 1 | 15 | 142 | colorless-green |
| Comparative Example 2 | 15 | 143 | colorless-green |

In view of the above: the optical material and lens comprising the polyurethane/spiropyran/zinc sulfide nano-composite microsphere made by the invention has the advantages of short photochromic response time and high spectral response sensitivity, etc.

## Claims

1. A photochromic nano-composite microsphere, **characterized in that**, the photochromic nano-composite microsphere is a polyurethane/spiropyran/zinc sulfide three-layer composite core-shell structure, its core is a zinc sulfide mesoporous nano-microsphere, its middle layer is a photochromic layer composed of the spiropyrans compound, and its shell is polyurethane; the outer diameter of the composite microsphere is 50-350 nm, wherein the diameter of the zinc sulfide mesoporous nano-microsphere is 30 ∼ 250 nm, the thickness of the middle layer is 5 ∼ 25 nm, and the thickness of the shell is 5 ∼ 25 nm.

2. The photochromic nano-composite microsphere according to claim 1, **characterized in that**, the zinc sulfide mesoporous nano-microsphere is a monodispersed zinc sulfide mesoporous nanosphere composed of the zinc sulfide nano-crystalline, and the middle layer is a nano-composite mesoporous structure formed by uniformly dispersing the spiropyrans compound nanoparticle on the surface or in the pore of the zinc sulfide mesoporous nanosphere.

3. The photochromic nano-composite microsphere according to claim 1 or 2, **characterized in that**, the spiropyrans compound is selected from the compound of formula (I), formula (II) or formula (III): 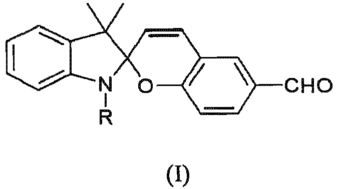 wherein R = C₁₆H₃₃ 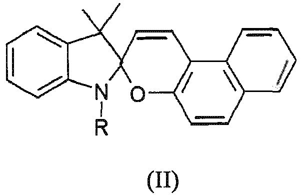 wherein R = C₁₆H₃₃

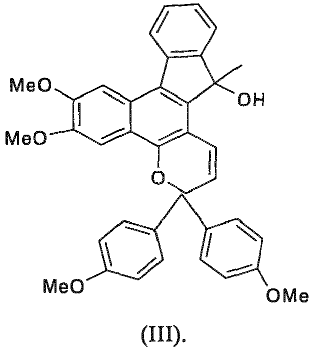

4. The photochromic nano-composite microsphere according to claim 1, **characterized in that**, the polyurethane is obtained by polymerization of an isocyanate monomer compound having two or more isocyanate groups with alcohols compound.

5. The photochromic nano-composite microsphere according to claim 4, **characterized in that**, the isocyanate monomer compound is selected from: at least one of toluene diisocyanate, diphenylmethane-4,4'-diisocyanate, 1,6-hexamethylene diisocyanate, m-xylylene diisocyanate, 1,5-naphthalene diisocyanate, methylcyclohexyl diisocyanate, dicyclohexylmethane diisocyanate, tetramethylxylylene diisocyanate, and isophorone diisocyanate;
the alcohols compound is at least one of n-butanol, polytetramethylene ether glycol, pentaerythritol, ethylene glycol, propylene glycol, butylene glycol, hexylene glycol, neopentyl glycol, and trihydroxymethyl propane.

6. A preparation method of the photochromic nano-composite microsphere according to any one of claims 1-5, **characterized in that**, the method includes the following steps:
a. Preparation of the spiropyrans-coated zinc sulfide nanosphere:
to ethylene glycol is added zinc nitrate, the mixture is uniformly stirred, sulfur powder is added, the temperature is elevated to 140-160 °C, and the mixture is reacted for 20-24 hours; the mixture is cooled to 40-60 °C, a mercaptoethanol stabilizer is dropwise added and the mixture is stirred; the spiropyrans compound is added, the mixture is vigorously stirred for 20-40 minutes and cooled to room temperature, the reaction solution gradually separates into layers and precipitates, and the precipitate is collected by filtration, washed, and dried to give the spiropyrans-coated zinc sulfide mesoporous nanosphere; wherein, the mass ratio of zinc nitrate to sulfur powder to mercaptoethanol to ethylene glycol is (0.3-0.6):(0.1-0.2):(4-7):(90-110), the mass ratio of zinc nitrate to the spiropyrans compound is 1:(0.5-1);
b. Preparation of the polyurethane/spiropyran/zinc sulfide photochromic nano-composite microsphere:
The spiropyrans-coated zinc sulfide mesoporous nanosphere prepared in step a and the isocyanate monomer compound are added into solvent, the mixture is uniformly stirred, and the alcohols compound and a dispersing agent are sequentially added; wherein the mass ratio of the spiropyrans-coated zinc sulfide mesoporous nanosphere to the isocyanate monomer compound to the alcohols compound to the dispersing agent to the solvent is (0.5-1.2):(0.5-1.2):(0.5-0.9):(1.2-2.2):100; the temperature is kept at 15-25 °C, and the mixture is prepolymerized for 45-60 minutes with stirring to give a prepolymer solution; chain extender and catalyst are added into the prepolymer solution, the mass ratio of the chain extender to the catalyst to the isocyanate monomer compound is (6-15):(0.1-0.3):(9-11), the mixture is heated to 45-90 °C to conduct chain extending polymerization reaction for 10-15 minutes to generate a polyurethane coating substance precipitate, and the precipitate is filtered, washed, and dried, to give the photochromic nano-composite microsphere having the polyurethane/spiropyran/zinc sulfide three-layer composite core-shell structure.

7. The preparation method of the photochromic nano-composite microsphere according to claim 6, **characterized in that**, the solvent is at least one of chloroform, acetone, propyl acetate, butyl acetate, ethyl acetate, dibutyl phthalate, and petroleum ether; the catalyst is dibutyltin dilaurate.

8. The preparation method of the photochromic nano-composite microsphere according to claim 6, **characterized in that**, the alcohols compound is a mixture of n-butanol and polytetramethylene ether glycol, wherein the mass ratio of n-butanol to polytetramethylene ether glycol is (1-3):(9-11).

9. The preparation method of the photochromic nano-composite microsphere according to claim 7, **characterized in that**, the dispersing agent is at least one of Tween 20, Tween 80, Span 20, Span 60, and Span 80;
the chain extender is at least one of ethylene glycol, propylene glycol, ethylene diamine, propylene diamine, and diethylamine;
preferably, the chain extender is a mixture of ethylene glycol and ethylene diamine, and the mass ratio of ethylene glycol to ethylene diamine is (2-3):(0.6-1.5).

10. A photochromic nano-composite microsphere, **characterized in that**, it is prepared by the method according to any one of claims 6-9.

11. A photochromic coating liquid, **characterized in that**, the coating liquid contains the photochromic nano-composite microsphere according to any one of claims 1-5 and claim 10.

12. The photochromic coating liquid according to claim 11, **characterized in that**, the coating liquid is composed of Component A and Component B according to the following parts and weight:
(1) Component A:
polythiocarbamate monomer 60-75 polyurethane/spiropyran/zinc sulfide photochromic nano-composite microsphere 2-7 diluent 7-15
wetting dispersing agent 1-2
leveling agent 0-1
(2) Component B:
isocyanate type curing agent 12-17
catalyst 0-1
defoamer 0-1.

13. The photochromic coating liquid according to claim 12, **characterized in that**, the polythiocarbamate monomer is at least one of 2,2'-dimercaptodiethylsulfide, 2,2'-dimercaptoethylthioethane, 2,3-dimercaptoethylthiopropanethiol, or 1,2,3-trimercaptoethyl-thiopropane;
the isocyanate type curing agent is at least one of toluene diisocyanate curing agent, diphenylmethane-4,4'-diisocyanate curing agent, 1,6-hexamethylene diisocyanate curing agent, m-xylylene diisocyanate curing agent, methylcyclohexyl diisocyanate curing agent, and isophorone diisocyanate curing agent;
the catalyst is dibutyltin dilaurate.

14. The photochromic coating liquid according to claim 12, **characterized in that**, the diluent is at least one of dichloromethane, butyl acetate, methyl acetate, ethanol, butanol, acetone, toluene, xylene, ethyl ether, and polytetramethylene ether glycol;
the wetting dispersing agent is Type BYK polyurethane exclusive wetting dispersing agent or Type F420 wetting dispersing agent;
the leveling agent is oily Type F300 leveling agent, Type F309 leveling agent, Type F309 leveling agent, or Type F320 leveling agent;
the defoamer is Type XPJ01F modified silicon oil defoamer.

15. The preparation method of the photochromic coating liquid according to any one of claims 12-14, the preparation method comprising the following operation steps:
(1) Component A: the polythiocarbamate monomer, the diluent, the polyurethane/ spiropyran/zinc sulfide photochromic composite microsphere, the wetting dispersing agent, and the leveling agent are added into a container respectively according to the ratio described, and the mixture is uniformly stirred at room temperature for 15-30 minutes to give Component A;
(2) Component B: the isocyanate type curing agent, the defoamer, and the catalyst are added into a container according to the ratio described, and the mixture is uniformly stirred at room temperature for 3-5 minutes to give Component B.

16. A photochromic coating, **characterized in that**, the coating comprises the photochromic nano-composite microsphere according to any one of claims 1-5.

17. A preparation method of a photochromic coating, **characterized in that**, the preparation method includes: the photochromic coating liquid according to any one of claims 12-14 is used to prepare the photochromic coating of optical substrate, the Component A and Component B are uniformly mixed according to the mass ratio of (70-100):(12-19), the viscosity thereof is adjusted by addition of a diluent; the mixture is then coated on the surface of an optical substrate to form a coating layer, which is cured through the light irradiation or heating to give the photochromic coating.

18. A photochromic optical material, **characterized in that**, it is composed of an optical resin substrate and the photochromic coating according to claim 16 coated on the surface thereof.

19. The photochromic optical material according to claim 18, **characterized in that**, the optical resin substrate is a thermoset plastic or thermoplastic plastic.

20. The photochromic optical material according to claim 18, **characterized in that**, the optical resin substrate is one of the polymethyl methacrylate resins, the propenyldiglycol carbonate resins, the polycarbonate resins, the carbamate resins, and the thioepoxy resins.

21. The photochromic optical material according to any one of claims 18-20, **characterized in that**, the optical resin substrate is the resin lens.

22. A preparation method of the photochromic optical material according to any one of claims 18-21, **characterized in that**, the method includes the following steps: the photochromic coating liquid is coated on the optical resin substrate by means of spin-coating, spray-coating or dip-coating to form the optical material having a photochromic coating on the surface.

23. The preparation method of the photochromic optical material according to claim 22, **characterized in that**, the method includes the following steps:
I. The coating liquid of Component A and Component B is formulated according to the method according to claim 15;
II. When coated, Component A and Component B are uniformly mixed according to the mass ratio of (70-100):(12-19), with the viscosity adjusted to 20 ∼ 350 cp (25 °C) by addition of the diluent, to give the photochromic coating liquid;
III. Pretreatment of the optical substrate: one or more of the following pretreatments are conducted to the optical resin substrate: chemical treatment with a basic aqueous solution or an acidic aqueous solution, grinding treatment, plasma treatment under different air pressures, corona discharge treatment, UV ozone treatment and hardening treatment;
IV. Coating: The optical resin substrate after the pretreatment is spin-coated, spray-coated or dip-coated with the photochromic coating liquid prepared in step II, to form a layer of photochromic coating on the substrate, i.e., the spiropyrans photochromic optical material is obtained.

24. The preparation method of the photochromic optical material according to claim 23, **characterized in that**, a first photochromic coating liquid having a viscosity of 60 ∼ 250 cp and a second photochromic coating liquid having a viscosity of 50 ∼ 200 cp are prepared respectively in the step II;
The dip-coating method is used in the step IV, and the specific operation thereof is as follow: the optical resin substrate after the pretreatment is immerged into the first photochromic coating liquid and soaked for 3-5 minutes at room temperature, and the optical resin substrate is then slowly pulled out of the coating liquid, transferred into an oven set at a temperature of 35-65 °C, and baked for 45-90 minutes to form the first photochromic coating; the optical resin substrate is then immerged into the second photochromic liquid and soaked at room temperature for 3-5 minutes, and the lens is then slowly pulled out of the coating liquid, transferred into an oven set at a temperature of 35-65 °C, and baked for 45-90 minutes, to form the second photochromic coating, and obtain the photochromic optical material.

25. The preparation method of the photochromic optical material according to any one of claims 22-24, **characterized in that**, the overall thickness of the photochromic coating is 5 ∼ 100 µm.

26. The preparation method of the photochromic optical material according to any one of claims 22-24, **characterized in that**, the method can also include the hardening and/or antireflective surface coating treatment of the surface of the optical material after the coating step IV.

## Patentansprüche

1. Photochrome Nanokomposit-Mikrokugel, **dadurch gekennzeichnet, dass** die photochrome Nanokomposit-Mikrokugel eine dreischichtige Polyurethan/Spiropyran/Zinksulfid-Komposit-Kern-Schalen-Struktur ist, ihr Kern eine mesoporöse Zink-Sulfid-Nanomikrokugel ist, ihre mittlere Schicht eine photochrome Schicht ist, die aus der Spiropyran-Verbindung besteht, und ihre Schale aus Polyurethan besteht und der Außendurchmesser der Komposit-Mikrokugel 50-350 nm beträgt, wobei der Durchmesser der mesoporösen Zink-Sulfid-Nanomikrokugel 30 ∼ 250 nm beträgt, die Dicke der mittleren Schicht 5 ∼ 25 nm beträgt und die Dicke der Schale 5 ∼ 25 nm beträgt.

2. Photochrome Nanokomposit-Mikrokugel nach Anspruch 1, **dadurch gekennzeichnet, dass** die mesoporöse Zinksulfid-Nanomikrokugel eine monodisperse mesoporöse Zinksulfid-Nanokugel ist, die aus dem nanokristallinen Zinksulfid besteht, und die mittlere Schicht eine mesoporöse Nanokomposit-Struktur ist, die durch gleichmäßiges Dispergieren des Spiropyran-Verbindung-Nanopartikels auf der Oberfläche oder in der Pore der mesoporösen Zinksulfid-Nanokugel gebildet wird.

3. Photochrome Nanokomposit-Mikrokugel nach Anspruch 1 oder 2, **dadurch gekennzeichnet, dass** die Spiropyran-Verbindung ausgewählt ist aus der Verbindung der Formel (I), der Formel (II) oder der Formel (III): 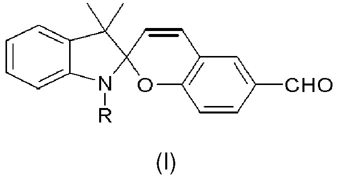 wobei R = C₁₆H₃₃ 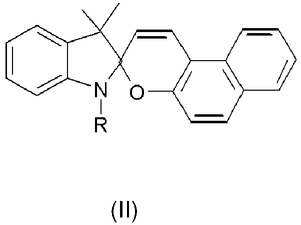 wobei R = C₁₆H₃₃

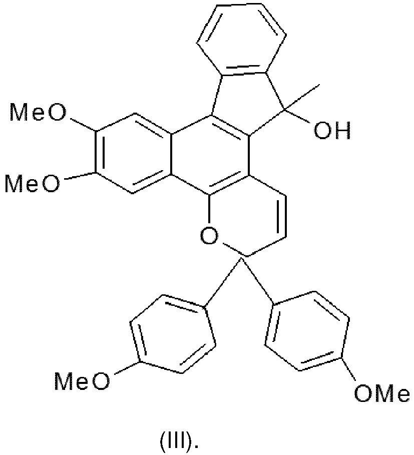

4. Photochrome Nanokomposit-Mikrokugel nach Anspruch 1, **dadurch gekennzeichnet, dass** das Polyurethan durch Polymerisation einer Isocyanatmonomerverbindung mit zwei oder mehr Isocyanatgruppen mit einer Alkoholverbindung erhalten wird.

5. Photochrome Nanokomposit-Mikrokugel nach Anspruch 4, **dadurch gekennzeichnet, dass**
die Isocyanat-Monomerverbindung ausgewählt ist aus: mindestens einem von Toluoldiisocyanat, Diphenylmethan-4,4'-diisocyanat, 1,6-Hexamethylendiisocyanat, m-Xylylendiisocyanat, 1,5-Naphthalindiisocyanat, Methylcyclohexyldiisocyanat, Dicyclohexylmethandiisocyanat, Tetramethylxylylendiisocyanat und Isophorondiisocyanat, und
die Alkoholverbindung mindestens eines ist von n-Butanol, Polytetramethylenetherglykol, Pentaerythrit, Ethylenglykol, Propylenglykol, Butylenglykol, Hexylenglykol, Neopentylglykol und Trihydroxymethylpropan .

6. Verfahren zur Herstellung der photochromen Nanokomposit-Mikrokugel nach einem der Ansprüche 1 - 5, **dadurch gekennzeichnet, dass** das Verfahren die folgenden Schritte umfasst:
a. Präparation der mit Spiropyranen beschichteten Zinksulfid-Nanokugel:
zu Ethylenglykol wird Zinknitrat hinzugefügt, die Mischung wird gleichmäßig gerührt, Schwefelpulver wird hinzugefügt, die Temperatur wird auf 140-160 °C erhöht und die Mischung wird 20 - 24 Stunden lang umgesetzt; die Mischung wird auf 40-60 °C abgekühlt, ein Mercaptoethanol-Stabilisator wird tropfenweise hinzugefügt und die Mischung wird gerührt; die Spiropyranverbindung wird zugegeben, die Mischung wird 20 - 40 Minuten lang kräftig gerührt und auf Raumtemperatur abgekühlt, die Reaktionslösung trennt sich allmählich in Schichten und fällt aus, und die Ausfällung wird durch Filtration gesammelt, gewaschen und getrocknet, um die mit Spiropyran beschichtete Zinksulfid mesoporöse Nanokugel zu ergeben; wobei das Massenverhältnis von Zinknitrat zu Schwefelpulver zu Mercaptoethanol zu Ethylenglykol (0,3 - 0,6) : (0,1 - 0,2) : (4 - 7) : (90-110) ist, das Massenverhältnis von Zinknitrat zu der Spiropyran-Verbindung 1 : (0,5 - 1) ist;
b. Herstellung der photochromen Nanokomposit-Mikrokugel aus Polyurethan/Spiropyran/Zinksulfid:
die in Schritt a hergestellte spiropyranbeschichtete mesoporöse Zinksulfid-Nanokugel und die Isocyanatmonomerverbindung werden in ein Lösungsmittel gegeben, die Mischung wird gleichmäßig gerührt, und die Alkoholverbindung und ein Dispergiermittel werden nacheinander zugegeben; wobei das Massenverhältnis der spiropyranbeschichteten mesoporösen Zinksulfid-Nanokugel zu der Isocyanatmonomerverbindung zu der Alkoholverbindung zu dem Dispergiermittel zu dem Lösungsmittel (0,5 - 1,2) : (0,5 - 1,2) : (0,5 - 0;9) : (1,2 - 2,2) : 100 ist; die Temperatur wird bei 15 - 25 °C gehalten, und die Mischung wird für 45 - 60 Minuten unter Rühren vorpolymerisiert, um eine Präpolymerlösung zu ergeben; Kettenverlängerer und Katalysator werden in die Präpolymerlösung gegeben, wobei das Massenverhältnis des Kettenverlängerers zum Katalysator zur Isocyanatmonomerverbindung (6 - 15) : (0,1 - 0,3) : (9 - 11) ist, die Mischung wird auf 45 - 90 °C erhitzt, um eine kettenverlängernde Polymerisationsreaktion für 10 - 15 Minuten durchzuführen, um eine Polyurethan-Beschichtungssubstanz-Ausfällung zu erzeugen, und die Ausfällung wird gefiltert, gewaschen und getrocknet, um die photochrome Nanokomposit-Mikrokugel mit der dreischichtigen Polyurethan/Spiropyran/Zinksulfid-Komposit-Kern-Schalen-Struktur zu erhalten.

7. Verfahren zur Herstellung der photochromen Nanokomposit-Mikrokugel nach Anspruch 6, **dadurch gekennzeichnet, dass** das Lösungsmittel mindestens eines von Chloroform, Aceton, Propylacetat, Butylacetat, Ethylacetat, Dibutylphthalat und Petrolether ist; der Katalysator ist Dibutylzinndilaurat.

8. Verfahren zur Herstellung der photochromen Nanokomposit-Mikrokugel nach Anspruch 6, **dadurch gekennzeichnet, dass** die Alkoholverbindung eine Mischung aus n-Butanol und Polytetramethylenetherglykol ist, wobei das Massenverhältnis von n-Butanol zu Polytetramethylenetherglykol (1 - 3) : (9 - 11) beträgt.

9. Verfahren zur Herstellung der photochromen Nanokomposit-Mikrokugel nach Anspruch 7, **dadurch gekennzeichnet, dass**
das Dispergiermittel mindestens eines von Tween 20, Tween 80, Span 20, Span 60 und Span 80 ist und
der Kettenverlängerer mindestens eines von Ethylenglykol, Propylenglykol, Ethylendiamin, Propylendiamin und Diethylamin ist;
vorzugsweise ist der Kettenverlängerer eine Mischung aus Ethylenglykol und Ethylendiamin, und das Massenverhältnis von Ethylenglykol zu Ethylendiamin beträgt (2 - 3) : (0,6 - 1,5).

10. Photochrome Nanokomposit-Mikrokugel, **dadurch gekennzeichnet, dass** sie nach dem Verfahren nach einem der Ansprüche 6 - 9 hergestellt ist.

11. Photochrome Beschichtungsflüssigkeit, **dadurch gekennzeichnet, dass** die Beschichtungsflüssigkeit die photochrome Nanokomposit-Mikrokugel nach einem der Ansprüche 1 - 5 und 10 enthält.

12. Photochrome Beschichtungsflüssigkeit nach Anspruch 11, **dadurch gekennzeichnet, dass** die Beschichtungsflüssigkeit aus der Komponente A und der Komponente B entsprechend den folgenden Teilen und dem folgenden Gewicht zusammengesetzt ist:
(1) Komponente A:
Polythiocarbamat-Monomer 60 - 75
Photochrome Nanokomposit-Mikrokugel aus Polyurethan/Spiropyran/Zinksulfid 2 - 7 Verdünnungsmittel 7 - 15
Benetzungsdispergiermittel 1 - 2
Nivelliermittel 0 - 1
(2) Komponente B:
Härtungsmittel vom Isocyanat-Typ 12 - 17
Katalysator 0 - 1
Entschäumer 0 - 1.

13. Photochrome Beschichtungsflüssigkeit nach Anspruch 12, **dadurch gekennzeichnet, dass**
das Polythiocarbamatmonomer mindestens eines von 2,2'-Dimercaptodiethylsulfid, 2,2'-Dimercaptoethylthioethan, 2,3-Dimercaptoethylthiopropanthiol oder 1,2,3-Trimercaptoethylthiopropan ist und
das Härtungsmittel vom Isocyanat-Typ mindestens eines von Toluoldiisocyanat-Härtungsmittel, Diphenylmethan-4,4'-diisocyanat-Härtungsmittel, 1,6-Hexamethylendiisocyanat-Härtungsmittel, m-Xylylendiisocyanat-Härtungsmittel, Methylcyclohexyldiisocyanat-Härtungsmittel und Isophorondiisocyanat-Härtungsmittel ist;
der Katalysator ist Dibutylzinndilaurat.

14. Photochrome Beschichtungsflüssigkeit nach Anspruch 12, **dadurch gekennzeichnet, dass**
das Verdünnungsmittel mindestens eines von Dichlormethan, Butylacetat, Methylacetat, Ethanol, Butanol, Aceton, Toluol, Xylol, Ethylether und Polytetramethylenetherglykol ist,
das Benetzungsdispergiermittel ein Polyurethan-Exklusiv-Benetzungsdispergiermittel von Typ BYK oder ein Benetzungsdispergiermittel vom Typ F420 ist und
das Nivelliermittel ein öliges Nivelliermittel vom Typ F300, ein Nivelliermittel vom Typ F309, ein Nivelliermittel vom Typ F309 oder ein Nivelliermittel vom Typ F320 ist;
der Entschäumer ist ein modifizierter Silikonölentschäumer vom Typ XPJ01F.

15. Verfahren zur Herstellung der photochromen Beschichtungsflüssigkeit nach einem der Ansprüche 12-14, wobei das Herstellungsverfahren die folgenden Arbeitsschritte umfasst:
(1) Komponente A: das Polythiocarbamat-Monomer, das Verdünnungsmittel, die photochrome Polyurethan/Spiropyran/Zinksulfid-Komposit-Mikrokugel, das Benetzungsdispergiermittel und das Nivelliermittel werden jeweils in einen Behälter gemäß dem beschriebenen Verhältnis zugegeben, und die Mischung wird bei Raumtemperatur 15 - 30 Minuten lang gleichmäßig gerührt, um Komponente A zu ergeben;
(2) Komponente B: das Härtungsmittel vom Isocyanat-Typ, der Entschäumer und der Katalysator werden in einen Behälter entsprechend dem beschriebenen Verhältnis zugegeben, und die Mischung wird bei Raumtemperatur 3 - 5 Minuten lang gleichmäßig gerührt, um die Komponente B zu erhalten.

16. Photochrome Beschichtung, **dadurch gekennzeichnet, dass** die Beschichtung die photochrome Nanokomposit-Mikrokugel nach einem der Ansprüche 1-5 umfasst.

17. Verfahren zur Herstellung einer photochromen Beschichtung, **dadurch gekennzeichnet, dass** das Herstellungsverfahren umfasst: die photochrome Beschichtungsflüssigkeit nach einem der Ansprüche 12 - 14 wird zur Herstellung der photochromen Beschichtung eines optischen Substrats verwendet, die Komponente A und die Komponente B werden gleichmäßig gemäß dem Massenverhältnis von (70 - 100) : (12 - 19) gemischt, die Viskosität davon wird durch Zugeben eines Verdünnungsmittels eingestellt; die Mischung wird dann auf die Oberfläche eines optischen Substrats aufgetragen, um eine Beschichtungsschicht zu bilden, die durch Lichtbestrahlung oder Erwärmung gehärtet wird, um die photochrome Beschichtung zu ergeben.

18. Photochromes optisches Material, **dadurch gekennzeichnet, dass** es aus einem optischen Harzsubstrat und der photochromen Beschichtung nach Anspruch 16 besteht, die auf dessen Oberfläche aufgetragen ist.

19. Photochromes optisches Material nach Anspruch 18, **dadurch gekennzeichnet, dass** das optische Harzsubstrat ein duroplasticher Kunststoff oder thermoplastischer Kunststoff ist.

20. Photochromes optisches Material nach Anspruch 18, **dadurch gekennzeichnet, dass** das optische Harzsubstrat eines der Polymethylmethacrylatharze, der Propenyldiglykolcarbonatharze, der Polycarbonatharze, der Carbamatharze und der Thioepoxyharze ist.

21. Photochromes optisches Material nach einem der Ansprüche 18 - 20, **dadurch gekennzeichnet, dass** das optische Harzsubstrat eine Harzlinse ist.

22. Verfahren zur Herstellung des photochromen optischen Materials nach einem der Ansprüche 18 - 21, **dadurch gekennzeichnet, dass** das Verfahren die folgenden Schritte umfasst: die photochrome Beschichtungsflüssigkeit wird auf das optische Harzsubstrat mittels Schleuderbeschichtung, Sprühbeschichtung oder Tauchbeschichtung aufgetragen, um das optische Material mit einer photochromen Beschichtung auf der Oberfläche zu bilden.

23. Verfahren zur Herstellung des photochromen optischen Materials nach Anspruch 22, **dadurch gekennzeichnet, dass** das Verfahren die folgenden Schritte umfasst:
I. die Beschichtungsflüssigkeit von Komponente A und Komponente B wird nach dem Verfahren nach Anspruch 15 formuliert;
II. beim Beschichten werden Komponente A und Komponente B entsprechend dem Massenverhältnis von (70 - 100) : (12 - 19) gleichmäßig gemischt, wobei die Viskosität durch Zugabe des Verdünnungsmittels auf 20 ∼ 350 cp (25 °C) eingestellt wird, um die photochrome Beschichtungsflüssigkeit zu erhalten;
III. Vorbehandeln des optischen Substrats: das optische Harzsubstrat wird einer oder mehreren der folgenden Vorbehandlungen unterzogen: chemische Behandlung mit einer basischen wässrigen Lösung oder einer sauren wässrigen Lösung, Schleifbehandlung, Plasmabehandlung unter verschiedenen Luftdrücken, Koronaentladungsbehandlung, UV-Ozonbehandlung und Härtungsbehandlung;
IV. Überzug: das optische Harzsubstrat wird nach der Vorbehandlung mit der in Schritt II vorbereiteten photochromen Beschichtungsflüssigkeit schleuderbeschichtet, sprühbeschichtet oder tauchbeschichtet, um eine photochrome Schicht auf dem Substrat zu bilden, d.h. es entsteht das photochrome optische Spiropyranmaterial.

24. Verfahren zur Herstellung des photochromen optischen Materials nach Anspruch 23, **dadurch gekennzeichnet, dass**
eine erste photochrome Beschichtungsflüssigkeit mit einer Viskosität von 60 ∼ 250 cp und eine zweite photochrome Beschichtungsflüssigkeit mit einer Viskosität von 50 ∼ 200 cp jeweils in Schritt II hergestellt werden;
im Schritt IV wird das Tauchbeschichtungsverfahren angewandt, dessen spezifische Funktionsweise wie folgt ist: das optische Harzsubstrat wird nach der Vorbehandlung in die erste photochrome Beschichtungsflüssigkeit eingetaucht und 3 - 5 Minuten lang bei Raumtemperatur eingeweicht, und das optische Harzsubstrat wird dann langsam aus der Beschichtungsflüssigkeit herausgezogen, in einen auf eine Temperatur von 35 - 63 °C eingestellten Ofen transferiert und 45 - 90 Minuten lang gebrannt, um die erste photochrome Beschichtung zu bilden; das optische Harzsubstrat wird dann in die zweite photochrome Flüssigkeit eingetaucht und bei Raumtemperatur 3 - 5 Minuten lang eingeweicht, und die Linse wird dann langsam aus der Beschichtungsflüssigkeit herausgezogen, in einen auf eine Temperatur von 35 - 65 °C eingestellten Ofen transferiert und 45 - 90 Minuten lang gebrannt, um die zweite photochrome Beschichtung zu bilden und das photochrome optische Material zu erhalten.

25. Herstellungsverfahren des photochromen optischen Materials nach einem der Ansprüche 22 - 24, **dadurch gekennzeichnet, dass** die Gesamtdicke der photochromen Beschichtung 5 ∼ 100 µm beträgt.

26. Verfahren zur Herstellung des photochromen optischen Materials nach einem der Ansprüche 22 - 24, **dadurch gekennzeichnet, dass** das Verfahren auch die Behandlung der Oberfläche des optischen Materials nach dem Beschichtungsschritt IV durch Härten und/oder Antireflexbeschichtung umfassen kann.

## Revendications

1. Microsphère nano-composite photochromique, cararactérisée en ce que la microsphère nano-composite photochromique est une structure composite de type coeur-enveloppe à trois couches polyuréthane/spiropyrane/sulfure de zinc, son coeur est une nano-microsphère mésoporeuse en sulfure de zinc, sa couche intermédiaire est une couche photochromique composé d'un composé spiropyrane, et son envelope est en polyurethane; le diamètre extérieur de la microsphere composite est 50-350 nm, où le diamètre de la nano-microsphère mésoporeuse en sulfure de zinc est 30 ∼ 250 nm, l'épaisseur de la couche intermédiaire est 5 ∼ 25 nm, et l'épaisseur de lenveloppe est 5 ∼ 25 nm.

2. Microsphère nano-composite photochromique selon la revendication 1, **caractérisée en ce que** la nano-microsphère mésoporeuse en sulfure de zinc est une nanosphère mésoporeuse monodispersée en sulfure de zinc composée de sulfure de zinc nano-cristallin, et la couche intermédiaire est une structure mésoporeuse nano-composite formée en dispersant uniformément des nanoparticules de composé spiropyrane à la surface ou dans les pores de la nanosphère mésoporeuse de sulfure de zinc.

3. Microsphère nano-composite photochromique selon la revendication 1 ou 2, **caractérisée en ce que** le composé spiropyrane est choisi parmi le composé de formule (I), de formule (II) ou de formule (III) : 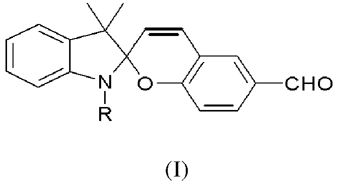 où R = C₁₆H₃₃ 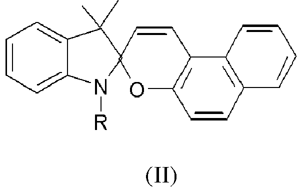 où R = C₁₆H₃₃

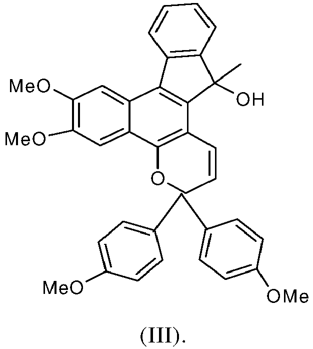

4. Microsphère nano-composite photochromique selon la revendication 1, **caractérisée en ce que** le polyurétahne est obtenu par polymérisation d'un composé monomère isocyanate ayant deux groupes isocyanate ou plus, avec un composé alccol.

5. Microsphère nano-composite photochromique selon la revendication 1, **caractérisée en ce que** le composé monomère isocyanate est choisi parmi au moins un de toluène diisocyanate, diphénylméthane-4,4'-diisocyanate, 1,6-hexaméthylène diisocyanate, m-xylylène diisocyanate, 1,5-naphtalène diisocyanate, méthylcyclohexyl diisocyanate, dicyclohexylméthane diisocyanate, tétraméthylxylylène diisocyanate, et isophorone diisocyanate;
le composé alcool est au moins un de n-butanol, polytétraméthylène éther glycol, pentaérythritol, éthylène glycol, propylène glycol, butylène glycol, hexylène glycol, néopentyl glycol, et trihydroxyméthyl propane.

6. Procédé de préparation de la microsphère nano-composite photochromique selon l'une quelconque des revendications 1-5, **caractérisé en ce que** le procédé comprend les étapes suivantes :
a. Préparation de nanosphères de sulfure de zinc revêtues de spiropyrane :
on ajoute du nitrate de zinc à de l'éthylène glycol, le mélange est agité uniformément, on ajoite de la poudre de soufre, on monte la temperature à 140-160 °C, et on fait réagir le mélange pendant 20-24 hours; le mélange est refroidi à 40-60 °C, un stabilisateur mercaptoéthanol est ajouté goutte à goutte et le mélange est agité; le composé spiropyrane est ajouté, le mélange est vigoureusement agité pendant 20-40 minutes et refroidi à la température ambiante, la solution réactionnelle se sépare progressivement en couches et précipite, et le précipitat est récupéré par filtration, lavé, et séché pour donner des nanosphères de sulfure de zinc revêtues de spiropyrane; où le rapport massique entre nitrate de zinc, poudre de soufre, mercaptoéthanol et éthylène glycol est (0,3-0,6):(0,1-0,2):(4-7):(90-110), et le rapport massique du nitrate de zinc au composé spiropyrane est 1:(0,5-1);
b. Préparation des microspheres nanocomposites photochromiques polyuréthane/spiropyrane/sulfure de zinc :
Les nanosphères mésoporeuses de sulfure de zinc revêtues de spiropyrane préparées à l'étape a et le composé monomère isocyanate sont ajoutés dans un solvant, le mélange est agité uniformément, et le composé alcool et un agent de dispersion sont ajoutés l'un après l'autre; où le rappot massique entre nanosphères mésoporeuses de sulfure de zinc revêtues de spiropyrane, composé monomère isocyanate, composé alcool, agent de dispersion et solvent est (0,5-1,2):(0,5-1,2):(0,5-0,9):(1,2-2,2):100; la température est mintenue à 15-25 °C, et le mélange est prépolymérisé pendant 45-60 minutes sous agitation pour donner une solution de prépolymère; un extenseur de chaîne et un catalyseur sont ajoutés à la solution de prépolymère, le rapport massique entre extenseur de chaîne, catalyseur et composé monomère isocynate est (6-15):(0,1-0,3):(9-11), le mélange est chauffé à 45-90 °C pour mener une réaction de polymérisation avec extension de chaîne pendant 10-15 minutes afin de générer un précipité de substance de revêtement polyurethane, et le précipité est filtré, lavé et séché pour donner des microspheres nanocomposites photochromiques ayant une structure cœur-enveloppe composite à trois couches polyuréthane/spiropyrane/sulfure de zinc.

7. Procédé de préparation de la microsphère nano-composite photochromique selon la revendication 6, **caractérisé en ce que** le solvent est au moins un parmi : chloroforme, acétone, acétate de propyle, acétate de butyle, acétate d'éthyle, phtalate de dibutyle, et éther de pétrole; et **en ce que** le catalyseur est le dilaurate de dibutylétain.

8. Procédé de préparation de la microsphère nano-composite photochromique selon la revendication 6, **caractérisé en ce que** le composé alcool est un mélange de n-butanol et de polytétraméthylène éther glycol, où le rapport massique du n-butanol au polytétraméthylène éther glycol est (1-3):(9-11).

9. Procédé de préparation de la microsphère nano-composite photochromique selon la revendication 7, **caractérisé en ce que** l'agent dispersant est au moins un de Tween 20, Tween 80, Span 20, Span 60, and Span 80;
l'extenseur de chaîne est au moins un de : éthylène glycol, propylène glycol, éthylène diamine, propylène diamine, et diéthylamine;
de préférence, l'extenseur de chaîne est un mélange d'éthylène glycol et d'éthylène diamine, et le rapport massqiue de l'éthylène glycol à l'éthylène diamine est (2-3):(0.6-1,5).

10. Microsphère nano-composite photochromique, **caractérisée en ce qu'**elle est préparée par le procédé selon l'une quelconque des revendications 6-9.

11. Liquide de revêtement photochromique, **caractérisé en ce que** le liquide de revêtement contient des microsphères nano-composites photochromiques selon l'une quelconque des revendications 1-5 et 10.

12. Liquide de revêtement photochromique selon la revendication 11, **caractérisé en ce que** le liquide de revêtement est composé du composant A et du composant B selon les proportions massique suivantes :
(1) Composant A :
monomère polythiocarbamate 60-75
microsphères nano-composites photochromiques polyuréthane/spiropyrane/sulfure de zinc 2-7
diluant 7-15
agent de dispersion mouillant 1-2
agent nivelant 0-1
(2) Composant B :
agent de durcissement de type isocyanate 12-17
catalyseur 0-1
agent anti-mousse 0-1.

13. Liquide de revêtement photochromique selon la revendication 12, **caractérisé en ce que** le monomère polythiocarbamate est au moins un de 2,2'-dimercaptodiéthylsulfure, 2,2'-dimercaptoéthylthioéthane, 2,3-dimercaptoéthylthiopropanethiol, ou 1,2,3-trimercaptoéthyl-thiopropane;
l'agent de durcissement de type isocyanate est au moins un parmi : un agent de durcissement toluène diisocyanate, un agent de durcissement diphénylmethane-4,4'-diisocyanate, un agent de durcissement 1,6-hexaméthylène diisocyanate, un agent de durcissement m-xylylène diisocyanate, un agent de durcissement méthylcyclohexyl diisocyanate, et un agent de durcissement isophorone diisocyanate;
et **en ce que** le catalyseur est le dilaurate de dibutylétain.

14. Liquide de revêtement photochromique selon la revendication 12, **caractérisé en ce que**
le diluant est au moins un de dichlorométhane, acétate de butyle, acétate de méthyle, éthanol, butanol, acétone, toluène, xylène, éther éthylique, et polytéetraméthylène éther glycol;
l'agent de dispersion mouillant est un agent de dispersion mouillant exclusif polyuréthane de type BYK, ou un agent de dispersion mouillant de type F420;
l'agent nivelant est un agent nivelant huileux de type F300, un agent nivelant de type F309, un agent nivelant de type F309, ou un agent nivelant de type F320;
l'agent anti-mousse est un anti-mousse huile de silicone modifiée de type XPJ01F.

15. Procédé de préparation du liquide de revêtement photochromique selon l'une quelconque des revendications 12-14, le procédé de préparation comprenant les étapes opératoires suivantes :
(1) Composant A: le monomère polythiocarbamate, le diluant, les microspheres nano-composites photochromiques polyuréthane/spiropyrane/sulfure de zinc, l'agent de dispersion mouillant, et l'agent nivelant sont ajoutés dans un réceptacle selon les rappports respectifs décrits, et le mélange est agité uniformément à température ambiante pendant 15-30 minutes pour donner le Composant A;
(2) Composant B: l'agent de durcissement de type isocyanate, l'agent anti-mousse et le catalyseur sont ajoutés dans un réceptacle selon le rapport décrit, et le mélange est agité uniformément à température ambiante pendant 3-5 minutes pour donner le Composant B.

16. Revêtement photochromique, **caractérisé en ce que** le revêtement comprend des microspheres nano-composites photochromiques selon l'une quiconque des revendications 1-5.

17. Procédé de préparation d'un revêtement photochromique, **caractérisé en ce que** le procédé de préparation comprend: le liquide de revêtement photochromique selon l'une quelconque des revendications 12-14 est utilisé pour préparer le revêtement photochromique d'un substrat optique, le Composant A et le Composant B sont mélangés uniformément selon le rapport massique (70-100):(12-19), la viscosité du mélange est ajustée par addition d'un diluant; le mélange est ensuite enduit sur la surface d'un substrat optique pour former une couche de revêtement qui est durcie par irradiation lumineuse ou chauffage pour donner le revêtement photochromique.

18. Matériau optique photochromique, **caractérisé en ce qu'**il est composé d'un substrat optique en résine et du revêtement photochromique selon la revendication 16 enduit sur la surface du substrat.

19. Matériau optique photochromique selon la revendication 18, **caractérisé en ce que** le substrat optique en résine est en plastique thermodurcissable ou en plastique thermoplastique.

20. Matériau optique photochromique selon la revendication 18, **caractérisé en ce que** le substrat optique en résine est un parmi des résines polyméthyl méthacrylate, des résines propényldiglycol carbonate, des résines polycarbonate, des résines carbamate, et des résines thioépoxy.

21. Matériau optique photochromique selon l'une quelconque des revendications 18-20, **caractérisé en ce que** le substrat optique en résine est une lentille en résine.

22. Procédé de préparation du matériau optique photochromique selon l'une quelconque des revendications 18-21, **caractérisé en ce que** le procédé comprend les étapes suivantes: le liquide de revêtement photochromique est enduit sur le substrat optique en résine par enduction centrifuge, pulvérisation ou enduction par trempage pour former un matériau optique ayant un revêtement photochromique à sa surface.

23. Procédé de préparation de matériau optique photochromique selon la revendication 22, **caractérisé en ce que** le procédé comprend les étapes suivantes:
I. le liquide de revêtement comprenant le Composant A et le Composant B est formulé selon le procédé de la revendication 15;
II. une fois formulés, le Composant A et le Composant B sont mélangés uniformément dans un rapport massique de (70-100):(12-19), la viscosité étant ajustée à 20 ∼ 350 cp (25 °C) par ajout de diluant, pour donner le liquide de revêtement photochromique;
III. Prétraitement du substrat optique: un ou plusieurs des traitements suivants sont mis en oeuvre sur le substrat optique en résine: traitement chimique avec une solution aqueuse basique ou une solution aqueuse acide, traitement par broyage, traitement plasma à différentes pressions, traitement par décharge corona, traitement UV à l'ozone et traitement durcissant;
IV. Enduction: à l'issue du prétraitement, le substrat optique en résine est enduit de liquide de revêtement photochromique préparé à l'étape II par enduction centrifuge, pulvérisation ou enduction par trempage, pour former une couche de revêtement photochromique sur le substrat, i.e., le matériau optique photochromique spiropyrane est obtenu.

24. Procédé de préparation de matériau optique photochromique selon la revendication 23, **caractérisé en ce qu'**un premier liquide de revêtement photochromique ayant une viscosité de 60 ∼ 250 cp et un second liquide de revêtement photochromique ayant une viscosité de 50 ∼ 200 cp sont respectivement préparés à l'étape II;
on utilise l'enduction par trempage à l'étape IV, et le mode opératoire spécifique est le suivant: après le prétraitement, le substrat optique en résine est immergé dans le premier liquide de revêtement photochromique et trempé pendant 3-5 minutes à temperature ambiante, et le substrat optique en résine est retiré lentement du liquide de revêtement, transféré dans une étuve réglée à une température de 35-65 °C, et cuit pendant 45-90 minutes, pour former le premier revêtement photochromique; le substrat optique en résine est ensuite immergé dans le second liquide de revêtement photochromique et trempé à température ambiante pendant 3-5 minutes, et la lentille est ensuite retirée lentement du liquide de revêtement, transférée dans une étuve réglée à une température de 35-65 °C, et cuite pendant 45-90 minutes, pour former le second revêtement photochromique et obtenir le matériau optique photochromique.

25. Procédé de préparation de matériau optique photochromique selon l'une quelconque des revendications 22-24, **caractérisé en ce que** l'épaisseur totale du revêtement photochromique est 5 - 100 µm.

26. Procédé de préparation de matériau optique photochromique selon l'une quelconque des revendications 22-24, **caractérisé en ce que** le procédé peut comprendre en outre le durcissement et/ou un traitement de revêtement de surface antiréfléchissant de la surface du matériau optique après l'étape d'enduction IV.
